# EUROPÄISCHE PATENTSCHRIFT

(11) **EP 1 852 102 B1**
(45) Veröffentlichungstag und Bekanntmachung des Hinweises auf die Patenterteilung: **05.10.2016**
(21) Anmeldenummer: 07008275.5
(22) Anmeldetag: 24.04.2007
(51) Int. Cl.: A61K 8/34, A61K 8/39, A61K 8/73, A61K 8/86, A61Q 15/00, A61K 8/33, A61K 8/37, A61Q 17/02, A61Q 17/04, A61Q 19/02, A61Q 19/04, A61Q 19/08, A61K 8/06, A61Q 19/00

(54) **Verfahren zur Herstellung von Öl-in-Wasser-Emulsionen zur Roll-on-Applikation**
Method for manufacturing oil in water emulsions for roll-on applications
Procédé de fabrication d'émulsions huile-dans-eau destinées à un applicateur à bille

(30) Priorität: 28.04.2006 DE 102006020380
(43) Veröffentlichungstag der Anmeldung: 07.11.2007
(73) Patentinhaber: Henkel AG & Co. KGaA, 40589 Düsseldorf (DE)
(72) Erfinder: Teckenbrock, Gertraud, 45549 Sprockhövel (DE); Heide, Barbara, 47809 Krefeld (DE)

(56) Entgegenhaltungen:
- EP-A- 1 584 370
- EP-A1- 0 265 087
- EP-A2- 0 659 404
- WO-A-00/21498
- DE-A1- 10 237 460
- GB-A- 2 013 085
- US-A- 5 356 612

## Beschreibung

Die vorliegende Anmeldung betrifft Verfahren zur Herstellung von Öl-in-Wasser-Emulsionen, die keine Mikroemulsionen sind und die insbesondere zur Applikation mit einem Rollapplikator geeignet sind und eine hohe Lagerstabilität, ein nicht-fettendes Hautgefühl und eine besonders schnelle Trocknungscharakteristik aufweisen, wobei die Emulsionen einen geringen Gehalt an Öl- oder Fettphase sowie mindestens ein Polysaccharid enthalten.

Es gibt zahlreiche Möglichkeiten, kosmetische Zusammensetzungen zur Haut- und Körperpflege auf die Haut aufzutragen. Cremes, Salben und Lotionen werden üblicherweise aus einem Tiegel, einer Tube oder einem Pumpspender entnommen und mit der Hand aufgetragen und verrieben. Formstabile Stiftmassen werden aus einem Stiftspender heraus über die Haut gestrichen, bis eine wirksame Menge aufgetragen ist. Auch Gele und Cremes können mit stiftähnlichen Dispensern, die mit einer Dispenseroberfläche über die Haut gestrichen werden, aufgetragen werden. Insbesondere für schweißhemmende und/oder desodorierende Zusammensetzungen für den Unterarmbereich wurden zahlreiche verschiedene Applikationsformen entwickelt, neben den bereits genannten vor allem die treibgashaltigen und treibgasfreien Sprays und die Roll-on-Zusammensetzungen. Bei letzteren wird eine leicht verdickte Flüssigkeit aus einem Vorratsbehälter über eine drehbar gelagerte Kugel durch Rollen über die Haut appliziert. Roll-on-Applikatoren werden zwar hauptsächlich für den Unterarmbereich eingesetzt, sind aber prinzipiell auch für die Pflege der Gesichtshaut und des Körpers geeignet. Für die Gesichtspflege dienen klein-dimensionierte Roll-on-Applikatoren insbesondere zum Auftragen höher konzentrierter Wirkstoff-Seren auf ausgewählte Problemzonen, z.B. Antifaften-Produkte für die Augenwinkel, die Stirn oder die Oberlippenregion, Anti-Akne-Produkte und Anti-Pickel-Produkte. Dies sorgt für einen (auch ökonomisch) effizienten Einsatz der wertvollen Wirkstoffe. Außerdem kann so der Einsatz von höher konzentrierten Wirkstoffen, die großflächig ein unangenehmes Hautgefühl erzeugen könnten (z.B. der Anti-Pickel-Wirkstoff Salicylsäure), lokal beschränkt werden. Gleichzeitig erlaubt der Applikator ein bequemes, zeitsparendes Auftragen. Zahlreiche kosmetische Wirkstoff sind wasserlöslich und ihre Freisetzung auf der Haut könnte durch Öl- und Fettbestandteile des Kosmetikums verzögert werden. Als rein wässrige Lösung wäre das Produkt allerdings kaum zu dosieren und damit für den Verbraucher nicht akzeptabel. Durch leichtes Andicken jedoch lässt sich eine solche Zusammensetzung bequem mit einem Roll-on-Applikator verwenden. Häufig werden polymere Verdickungsmittel eingesetzt. Nachteilig hierbei ist, dass die meisten polymeren Verdickungsmittel in den erforderlichen Konzentrationen ein sehr klebriges Hautgefühl erzeugen. Darüber hinaus weisen viele dieser Verdicker keine zusätzlichen kosmetischen Pflegeeffekte auf. Eine vorteilhafte Alternative hierzu stellen Emulsionen mit einem geringen Öl- und Fettgehalt dar. Die Emulsionsbildung führt auch ohne Polymerverdicker zu einem Anstieg der Viskosität. Der Öl- und Fettanteil der Emulsion entfaltet darüber hinaus eine hautpflegende Wirkung.
Emulsionen sind, im Gegensatz zu Mikroemulsionen, thermodynamisch instabil. Die thermodynamisch stabilen Mikroemulsionen können meist nur durch einen relativ hohen Emulgatorgehalt stabilisiert werden. Ein hoher Gehalt an Emulgatoren kann jedoch im ungünstigsten Fall hautreizend wirken und wird daher möglichst vermieden. Außerdem bilden sich Mikroemulsionen häufig nur in einem sehr engen Mischungsbereich der einzelnen Komponenten. Bei kosmetischen Zusammensetzungen mit mehreren Bestandteilen kann es daher entwicklungstechnisch mitunter sehr schwierig, geeignete Mikroemulsionsbereiche einzustellen. Emulsionen sind für eine gewisse Zeit stabil, weil die Koaleszenz der dispergierten Tröpfchen kinetisch gehemmt ist. Diese kinetische Hemmung kann aufgehoben werden durch Lagerung bei hohen Temperaturen (relevant insbesondere für die Produktion und Vermarktung in warmen Ländern) oder bei Lagerung unter größeren Temperaturschwankungen (z. B. in unzureichend klimatisierten Verkaufsräumen, beim Transport über weitere Strecken). Auch die hohe Salzkonzentration in Antitranspirant-Zusammensetzungen, bedingt durch die relativ hohe Konzentration an schweißhemmenden Wirkstoffen, kann die Emulsionsdestabilisierung begünstigen (z. B. durch Aussalzeffekte).
Bei Roll-on-Emulsionen mit einem typischerweise hohen Wasseranteil kann das feuchte Hautgefühl direkt nach der Applikation vom Verbraucher als unangenehm wahrgenommen werden.

Aus EP 270 328 sind schweißhemmende Öl-in-Wasser-Emulsionen bekannt, die mit einem hohen Gehalt an Polysacchariden eine Verkapselung der enthaltenen Parfümöle erzielen. Der hohe Polysaccharidgehalt kann sich aber ungünstig vor allem auf das Hautgefühl auswirken. Außerdem kann der hohe Polysaccharidgehalt auch die Lagerstabilität der Emulsionen, insbesondere bei höheren Lagertemperaturen von 45 °C und darüber, beeinträchtigen.
Aus US 4499069 sind schweißhemmende Öl-in-Wasser-Emulsionen bekannt, die etwa 22 Gew.-% einer Ölphase, umfassend flüchtige Siliconöle und PPG-15-Stearylether, Steareth-2, Steareth-21 sowie 2 Gew.-% Aluminiumstärkeoctenylsuccinat enthalten. Diese Emulsionen sind zwar als lagerstabil bezeichnet, allerdings ist auch angegeben, dass sie nach 4 Wochen Lagerung bei 45 °C ein Aufrahmen der dispergierten Phase zeigen. Dieses Stabilitätsverhalten ist für heutige Verbraucheransprüche nicht mehr ausreichend.
Aus US 6261543 sind schweißhemmende Öl-in-Wasser-Emulsionen bekannt, die etwa 6,5 - 10 Gew.-% einer Öl- und/oder Fettphase, ein Gemisch aus hydrophilen und lipophilen Emulgatoren sowie 1 Gew.-% einer amphoteren oder kationischen Stärke enthalten. Ein entsprechendes Vergleichsbeispiel mit einer nichtionischen Stärke wurde als bei 50 °C nicht lagerstabil angegeben.
Keines dieser Dokumente offenbart ein Herstellverfahren gemäß der vorliegenden Anmeldung.

Eine Aufgabe der vorliegenden Erfindung war es, ein Herstellverfahren für eine, beispielsweise schweißhemmende, Öl-in-Wasser-Emulsion mit verbesserter Lagerstabilität, insbesondere verlängerter Lagerstabilität bei Temperaturen von 40 °C und darüber, bereitzustellen. Eine weitere Aufgabe der vorliegenden Erfindung war es, ein Herstellverfahren für eine schweißhemmende, beispielsweise schweißhemmende, Öl-in-Wasser-Emulsion mit einem nicht-fettenden Hautgefühl bereitzustellen. Eine weitere Aufgabe der vorliegenden Erfindung war es, ein Herstellverfahren für eine, beispielsweise schweißhemmende, Öl-in-Wasser-Emulsion bereitzustellen, die möglichst schnell auf der Haut trocknet.
Überraschend wurde gefunden, dass nach einem Herstellverfahren, wie es in den Patentansprüchen dieser Anmeldung beansprucht ist, Öl-in-Wasser-Emulsionen mit einem Anteil an Öl- oder Fettphase von maximal 15 Gew.-%, enthaltend ausgewählte Olkomponenten in Kombination mit einem, vorzugsweise geringen, Anteil an mindestens einem Polysaccharid, hergestellt werden können, die auch bei hohen Temperaturen von 45 °C und darüber mehrere Wochen lang lagerstabil sind und die darüber hinaus nach dem Auftrag auf die Haut eine Trockengeschwindigkeit aufweisen, die vom Anwender als gegenüber dem Stand der Technik deutlich verkürzt wahrgenommen wird.

Ein erster Gegenstand der vorliegenden Erfindung ist daher ein Verfahren zur Herstellung einer Öl-in-Wasser-Emulsion, die keine Mikroemulsion darstellt und die enthält:
a) 0,5 - 15 Gew.-% Öl- oder Fettphase, umfassend mindestens eine bei 20 °C flüssige Ölkomponente, ausgewählt aus
   i) linearen und verzweigten gesättigten ein- oder mehrwertigen C₃ - C₃₀-Alkanolen, die mit mindestens einer Propylenoxid-Einheit pro Molekül verethert sind,
   ii) Propylenglycolmonoestern von verzweigten gesättigten C₆ - C₃₀-Alkancarbonsäuren,
   iii) verzweigten gesättigten C₁₀ - C₃₀-Alkanolen,
b) mindestens 60 Gew.-% Wasser,
c) 0,00001 - 38 Gew.-% mindestens eines kosmetischen Wirkstoffes,
d) mindestens ein Polysaccharid,
e) gegebenenfalls mindestens einen nichtionischen Emulgator mit einem HLB-Wert im Bereich von 3 - 6,
f) gegebenenfalls mindestens einen nichtionischen Emulgator mit einem HLB-Wert im Bereich von 12 -18,
g) mindestens einen Duftstoff in der Öl- oder Fettphase,
h) gegebenenfalls weitere Öl- oder Fettkomponenten,
wobei alle Mengenangaben auf das Gesamtgewicht der Emulsion bezogen sind und das Verfahren folgende Schritte umfasst:
(A) Erwärmung einer Portion X1 der gesamten Wassermenge (Phase X) und der Ölkomponente/n i) - iii) und der/die gegebenenfalls enthaltene/n Emulgator/en sowie ggf. weitere Öl- oder Fettkomponenten (Phase Y) getrennt voneinander auf eine Temperatur zwischen 70 - 80° C,
(B) anschließend langsames Vermischen der Portion X1 von Phase X mit der Phase Y,
(C) anschließend Homogenisierung,
(D) anschließend Rühren bei niedriger Drehzahl,
(E) anschließend Abkühlen des Ansatzes auf eine Temperatur im Bereich von 40 - 50° C,
(F) anschließend Bereitstellen einer Portion X2 der gesamten Wassermenge, die eine Portion c1 des kosmetischen Wirkstoffs c) enthält, sofern dieser ausreichend temperaturstabil ist, bei einer Temperatur im Bereich von 40 - 50° C,
(G) anschließend langsames Vermischen der Portion (X2 + c1) mit dem gesamten Ansatz bei einer Temperatur im Bereich von 40 - 50° C,
(H) anschließend Homogenisierung,
(I) anschließend langsames Vermischen der noch verbleibenden Portion X3 des Wassers, wobei der Ansatz eine Temperatur im Bereich von 30 - 40° C hat,
(J) anschließend Homogenisierung,
(K) anschließend Zugabe des Polysaccharids und des Duftstoffes sowie ggf. des/der kosmetischen Wirkstoffs/Wirkstoffe c), sofern diese/r bei 40 - 50° C nicht ausreichend temperaturstabil ist/sind sowie ggf. weiterer Zusatzstoffe wie Konservierungsmittel, pH-Regulatoren etc.,
(L) anschließend Homogenisierung,
(M) abschließend Abkühlen auf 25 °C unter langsamem Rühren, wobei
   - die Verfahrensschritte (F), (G) und (H) einmal, zweimal, dreimal oder mehr als dreimal wiederholt werden, bevor Schritt (I) erfolgt,
   - die Portion X1 ein Fünftel bis ein Drittel der gesamten Wassermenge beträgt,
   - der Homogenisierschritt (C) bei niedriger Scherrate im Bereich von 1000 - 2500 Umdrehungen des Rührelementes pro Minute erfolgt und
   - die Homogenisierschritte (H), (J) und (L) bei hoher Scherrate im Bereich von 3000 - 6000 Umdrehungen des Rührelementes pro Minute erfolgen.

Ein erfindungsgemäß bevorzugtes Verfahren ist dadurch gekennzeichnet, dass in Schritt (B) die Wasserphase X zur öl- und fetthaltigen Phase (Y) gegeben wird.

Ein anderes erfindungsgemäß bevorzugtes Verfahren ist dadurch gekennzeichnet, dass in Schritt (B) die öl- und fetthaltige Phase (Y) zur Wasserphase X gegeben wird.

Weitere erfindungsgemäß bevorzugte Verfahren sind dadurch gekennzeichnet, dass der mindestens eine kosmetische Wirkstoff c) ausgewählt ist aus:
- schweißhemmenden Wirkstoffen,
- desodorierenden Wirkstoffen,
- Monomeren, Oligomeren und Polymeren von Aminosäuren, N-C₂-C₂₄-Acylaminosäuren, den Estern und/oder den physiologisch verträglichen Salzen dieser Substanzen,
- DNA- oder RNA-Oligonucleotiden,
- natürlichen Betainverbindungen,
- Vitaminen, Provitaminen und Vitaminvorstufen der Gruppen A, B, C, E, H und K und den Estern der vorgenannten Substanzen,
- α-Hydroxycarbonsäuren, α-Ketocarbonsäuren, β-Hydroxycarbonsäuren und deren Ester-, Lacton- oder Salzform,
- Flavonoiden und Flavonoid-reichen Pflanzenextrakten,
- Isoflavonoiden und Isoflavonoid-reichen Pflanzenextrakten,
- Polyphenolen und Polyphenol-reichen Pflanzenextrakten,
- Ubichinon und Ubichinol sowie deren Derivaten,
- Silymarin,
- Ectoin,
- Repellentien,
- anorganischen und organischen UV-Filtersubstanzen,
- selbstbräunenden Wirkstoffen,
- hautaufhellenden Wirkstoffen,
- hautberuhigenden Wirkstoffen,
- feuchtigkeitsspendenden Wirkstoffen,
- sebumregulierenden Wirkstoffen.

Ein weiterer Gegenstand der vorliegenden Anmeldung sind Öl-in-Wasser-Emulsionen, die durch die erfindungsgemäßen Herstellverfahren erhältlich sind.

Die erfindungsgemäß erhältlichen Öl-in-Wasser-Emulsionen zeichnen sich durch einen Gehalt an mindestens einem Polysaccharid aus. Überraschend wurde festgestellt, dass der Polysaccharid-Gehalt das Trocknen der Emulsion auf der Haut beschleunigt Gegenüber einer Polysaccharid-freien Emulsion werden erfindungsgemäße Emulsionen von den Testpersonen als auf der Haut schneller trocknend wahrgenommen.

Unter erfindungsgemäß bevorzugten Polysacchariden werden sowohl nicht-modifizierte Polysaccharide, wie beispielsweise Xanthan oder Stärke, als auch chemisch modifizierte Polysaccharid-Derivate, wie beispielsweise Aluminiumstärkeoctenylsuccinat oder Hydroxypropylmethylcellulose, als auch physikalisch modifizierte Polysaccharide, beispielsweise eine durch thermische Behandlung vorverkleisterte Stärke, verstanden.
Erfindungsgemäß besonders bevorzugte Polysaccharide sind ausgewählt aus Stärken, insbesondere aus Mais, Kartoffeln und Weizen, deren Bestandteilen wie Amylose und Amylopektin, Stärkehydrolysaten und Stärkeabbauprodukten, wie Maltodextrin, den physikalisch oder chemisch modifizierten Stärkederivaten, insbesondere den anionischen Stärkederivaten Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken und Natriumstärkeglycolat, Cellulose, den chemisch modifizierten Cellulosederivaten Methylcellulose, Hydroxypropylcellulose, Hydroxyethylcellulose, Hydroxypropylmethylcellulose, Hydroxypropylethylcellulose, Hydroxyethylmethylcellulose und Carboxymethylcellulose. Polysaccharide, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums und Agar-Agar, können ebenfalls enthalten sein, sind aber weniger bevorzugt. In einer besonders bevorzugten Ausführungsform sind die erfindungsgemäß hergestellten Zusammensetzungen frei von Polysaccharid-Gums. In einer weiteren besonders bevorzugten Ausführungsform sind die erfindungsgemäß hergestellten Zusammensetzungen frei von Guar-Gum, Xanthan-Gum, Dehydroxanthan Gum, Alginate, insbesondere Natriumalginat, Gummi arabicum, Karaya-Gummi, Carrageenane, Johannisbrotkernmehl, Leinsamen-Gums und Agar-Agar.

Besonders bevorzugte erfindungsgemäß erhältliche Öl-in-Wasser-Emulsionen sind dadurch gekennzeichnet, dass das mindestens eine Polysaccharid ausgewählt ist aus anionischen und nichtionischen Polysacchariden sowie Mischungen hiervon.

Weitere besonders bevorzugte erfindungsgemäß erhältliche Öl-in-Wasser-Emulsionen sind dadurch gekennzeichnet, dass das anionische Polysaccharid ausgewählt ist aus Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken, Natriumstärkeglycolat sowie Mischungen hiervon. Ein erfindungsgemäß außerordentlich bevorzugtes anionisches Polysaccharid ist Aluminiumstärkeoctenylsuccinat.

Weitere besonders bevorzugte erfindungsgemäß erhältliche Öl-in-Wasser-Emulsionen sind dadurch gekennzeichnet, dass das nichtionische Polysaccharid ausgewählt ist aus Stärken, Stärkehydrolysaten, Cellulose, Methylcellulose, Hydroxypropylcellulose, Hydroxyethylcellulose, Hydroxypropylmethylcellulose, Hydroxypropylethylcellulose, Hydroxyethylmethylcellulose sowie Mischungen hiervon.

Weitere besonders bevorzugte erfindungsgemäß erhältliche Öl-in-Wasser-Emulsionen sind dadurch gekennzeichnet, dass das mindestens eine Polysaccharid in einer Gesamtmenge von 0,01 - 1,0 Gew.-%, bevorzugt 0,05 - 0,5 und besonders bevorzugt 0,09 - 0,2 Gew.-%, jeweils bezogen auf das Gesamtgewicht der Emulsion, enthalten ist. Es war besonders überraschend, dass schon und gerade mit relativ geringen Mengen an Polysaccharid ein schnelleres Trockengefühl der Emulsion auf der Haut erzielt werden konnte.

Kennzeichnend für das erfindungsgemäße Verfahren ist, dass das mindestens eine Polysaccharid zur fertigen Emulsion gegeben wird. Die Verfahrensschritte (A) - (E) der eigentlichen Emulsions-bildung ergeben auch in Abwesenheit von Polysacchariden stabile Öl-in-Wasser-Emulsionen am Ende des gesamten Verfahrens.

Die erfindungsgemäß erhältlichen Öl-in-Wasser-Emulsionen zeichnen sich gegenüber dem Stand der Technik weiterhin durch einen geringen Anteil an einer Öl- oder Fettphase von 0,5 - 15 Gew.-%, bezogen auf das Gewicht der gesamten Emulsion, aus. Der geringe Anteil an dispergierter Öl- oder Fettphase führt zu einem verbesserten, nicht-fettenden Hautgefühl. Weiterhin stellen die erfindungsgemäß erhältlichen Emulsionen eine hervorragende, nicht-komedogene Basis insbesondere für kosmetische und dermatologische Wirkstoffe dar, die zur Behandlung fettiger, unreiner Haut und/oder Akne-Haut bestimmt sind. Weiterhin stellen die erfindungsgemäß erhältlichen Emulsionen eine hervorragende Basis für Sonnenschutzzusammensetzungen dar, da gerade der Fett- und Emulgatorgehalt häufig für Unverträglichkeitsreaktionen solcher Zusammensetzungen unter Einwirkung des Sonnenlichtes verantwortlich ist. Mit den erfindungsgemäß erhältlichen Emulsionen lässt sich das Risiko für Unverträglichkeitsreaktionen deutlich minimieren. Weiterhin stellen die erfindungsgemäß erhältlichen Emulsionen eine hervorragende Basis für selbstbräunende Zusammensetzungen dar, deren Wirkstoffe, insbesondere Dihydroxyaceton, in bekannten Emulsionen nur schwierig zu stabilisieren sind, da sie mit zahlreichen, üblicherweise eingesetzten Emulsionsbestandteilen undefinierte Reaktionen eingehen, die zur Deaktivierung des Wirkstoffes und zur Verfärbung des Kosmetikums führen. Weiterhin bieten die erfindungsgemäß erhältlichen Emulsionen auch ökonomische Vorteile.
Allerdings lassen sich derartige Emulsionen üblicherweise nicht mit einer Viskosität herstellen, die für eine Applikation mit einem Kugel- oder Roll-on-Applikator erforderlich ist. Eine besondere Herausforderung der vorliegenden Erfindung war es daher, kosmetische Öl-in-Wasser-Emulsionen mit einem Anteil an einer Öl- oder Fettphase von 0,5 - 15, bevorzugt 1 - 10 Gew.-%, besonders bevorzugt 1,5 - 6,5 Gew.-%, jeweils bezogen auf das Gewicht der gesamten Emulsion, und einer für die Applikation als Roll-on ausreichenden Viskosität herzustellen. Die Öl- oder Fettphase umfasst erfindungsgemäß neben der mindestens einen bei 20 °C flüssigen Ölkomponente, die ausgewählt ist aus linearen und verzweigten gesättigten ein- oder mehrwertigen C₃ - C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, mindestens einen Duftstoff. Darüber hinaus können auch bei 20 °C feste oder pastöse Fettkomponenten enthalten sein. Die Emulgatoren zählen definitionsgemäß nicht zur Öl- oder Fettphase.
Zu den bei 20 °C flüssigen Ölkomponenten, die ausgewählt sind aus linearen und verzweigten gesättigten ein- oder mehrwertigen C₃ - C₃₀-Alkanolen, die mit mindestens einer Propylenoxid-Einheit pro Molekül verethert sind, zählen bevorzugt Propanol, Glycerin, Propylenglycol, Butanol, Butandiol, Pentanol, Caprinalkohol, Caprylalkohol, Caprylylalkohol, Laurylalkohol, Tridecylalkohol, Myristylalkohol, Palmitylalkohol, Cetylalkohol, Stearylalkohol, Arachidylalkohol und Behenylalkohol, die mit 1, 2, 3, 4, 5, 6, 7, 8, 9, 10, 11, 12, 13, 14, 15, 16, 17, 18, 19, oder 20 Propylenoxideinheiten verethert sind.

Bevorzugte erfindungsgemäß erhältliche Öl-in-Wasser-Emulsionen sind dadurch gekennzeichnet, dass die Ölkomponente i) ausgewählt ist aus Anlagerungsprodukten von mindestens 6 Propylenoxid-Einheiten pro Molekül an ein- oder mehrwertige C₃₋₃₀-Alkanole, insbesondere an Butanol, Butandiol, Myristylalkohol und Stearylalkohol.

Besonders bevorzugte erfindungsgemäß erhältliche Öl-in-Wasser-Emulsionen sind dadurch gekennzeichnet, dass die Ölkomponente i) ausgewählt ist aus PPG-3-Myristylether (Handelsprodukt Witconol^{®} APM), PPG-13-Butylether, PPG-14-Butylether (Handelsprodukt Ucon Fluid^{®} AP), PPG-9-Butylether (Handelsprodukt Breox^{®} B25), PPG-10-Butandiol (Handelsprodukt Macol^{®} 57) und PPG-15-Stearylether (Handelsprodukt Arlamol^{®} E), sowie Mischungen hiervon.

Weitere bevorzugte erfindungsgemäß erhältliche Öl-in-Wasser-Emulsionen sind dadurch gekennzeichnet, dass die Ölkomponente ii) ausgewählt ist aus Propylenglycolmonoestern von verzweigten gesättigten C₆ - C₃₀-Alkancarbonsäuren.
Erfindungsgemäß besonders bevorzugte Ölkomponenten ii) sind ausgewählt aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon.

Weitere bevorzugte erfindungsgemäß erhältliche Öl-in-Wasser-Emulsionen sind dadurch gekennzeichnet, dass die Ölkomponente iii) ausgewählt ist aus verzweigten gesättigten C₁₀ - C₃₀-Alkanolen. Erfindungsgemäß besonders bevorzugte Ölkomponenten iii) sind ausgewählt aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, Guerbetalkoholen, insbesondere 2-Hexyldecyllaurat, 2-Hexyldecanol und 2-Octyldodecanol. sowie Mischungen hiervon.

Weitere bevorzugte erfindungsgemäß erhältliche Öl-in-Wasser-Emulsionen sind dadurch gekennzeichnet, dass die mindestens eine Ölkomponente, ausgewählt aus den oben genannten Gruppen i), ii) und iii), in einer Gesamtmenge von 0,1 - 6,5 Gew.-%, bevorzugt 0,3 - 5 Gew.-%, besonders bevorzugt 0,5 - 3 Gew.-% und außerordentlich bevorzugt 1 - 2 Gew.-%, jeweils bezogen auf das Gesamtgewicht der Emulsion, enthalten ist.

Weitere bevorzugte erfindungsgemäß erhältliche Öl-in-Wasser-Emulsionen sind dadurch gekennzeichnet, dass die Öl- oder Fettphase in einer Gesamtmenge von 0,7 - 12 Gew.-%, bevorzugt 1 - 6,5 Gew.-%, besonders bevorzugt 1,5 - 3 Gew.-% und außerordentlich bevorzugt 2 - 2,5 Gew.-%, jeweils bezogen auf das Gesamtgewicht der Emulsion, enthalten ist.

Überraschend wurde festgestellt, dass die Lagerstabilität der erfindungsgemäß erhältlichen ÖI-in-Wasser-Emulsionen durch den Zusatz von mindestens einem nichtionischen Emulgator mit einem HLB-Wert im Bereich von 3 - 6 weiter gesteigert werden kann. Derartige lipophile Emulgatoren stabilisieren normalerweise Wasser-in-Öl-Emulsionen.
Weitere bevorzugte erfindungsgemäß erhältliche Öl-in-Wasser-Emulsionen sind dadurch gekennzeichnet, dass mindestens ein nichtionischer Emulgator mit einem HLB-Wert im Bereich von 3 - 6 enthalten ist.
Erfindungsgemäß bevorzugte nichtionische Emulgatoren mit einem HLB-Wert im Bereich von 3 - 6 sind ausgewählt aus linearen gesättigten und ungesättigten C₁₂ - C₃₀-Alkanolen, die mit 1 - 4 Ethylenoxid-Einheiten pro Molekül verethert sind.
Besonders bevorzugte nichtionische Emulgatoren mit einem HLB-Wert im Bereich von 3 - 6 sind ausgewählt aus Steareth, Ceteth, Myristeth, Laureth, Trideceth, Arachideth und Beheneth mit jeweils 1 - 4 Ethylenoxid-Einheiten pro Molekül. Außerordentlich bevorzugt sind Steareth-1, Steareth-2, Steareth-3, Ceteth-1, Ceteth-2, Ceteth-3, Myristeth-1, Myristeth-2, Laureth-1 Beheneth-2, Beheneth-3 und Beheneth-4, insbesondere Steareth-2.

Weitere bevorzugte erfindungsgemäß erhältliche Öl-in-Wasser-Emulsionen sind dadurch gekennzeichnet, dass mindestens ein nichtionischer Emulgator mit einem HLB-Wert im Bereich von 3 - 6 in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gewicht der gesamten Emulsion, enthalten ist.

Weiterhin wurde überraschend festgestellt, dass die Lagerstabilität der erfindungsgemäß erhältlichen Öl-in-Wasser-Emulsionen durch den Zusatz von mindestens einem nichtionischen Emulgator mit einem HLB-Wert im Bereich von 12 - 18 weiter gesteigert werden kann. Weitere erfindungsgemäß bevorzugte Öl-in-Wasser-Emulsionen sind dadurch gekennzeichnet, dass mindestens ein nichtionischer Emulgator mit einem HLB-Wert im Bereich von 12 - 18 enthalten ist. Erfindungsgemäß bevorzugte nichtionische Emulgatoren mit einem HLB-Wert im Bereich von 12 - 18 sind ausgewählt aus linearen gesättigten und ungesättigten C₁₂ - C₂₄-Alkanolen, die mit 7 - 40 Ethylenoxid-Einheiten pro Molekül verethert sind. Besonders bevorzugte nichtionische Emulgatoren mit einem HLB-Wert im Bereich von 12 -18 sind ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-15, Steareth-20, Steareth-21, Arachideth-20, Arachideth-21, Beheneth-20, Beheneth-21, Ceteth-20, Ceteth-30, Ceteth-15 und Myristeth-15.

Weitere bevorzugte erfindungsgemäß erhältliche Öl-in-Wasser-Emulsionen sind dadurch gekennzeichnet, dass mindestens ein nichtionischer Emulgator mit einem HLB-Wert im Bereich von 12 - 18 in einer Gesamtmenge von 1 - 2 Gew.-%, bevorzugt 1,2 - 1,8 Gew.-% und besonders bevorzugt 1,5 -1,7 Gew.-%, jeweils bezogen auf das Gewicht der gesamten Emulsion, enthalten ist.

Weitere erfindungsgemäß bevorzugte Öl-in-Wasser-Emulsionen sind dadurch gekennzeichnet, dass als nichtionischer Emulgator mit einem HLB-Wert im Bereich von 3 - 6 Steareth-2 in Kombination mit Steareth-21 als nichtionischem Emulgator mit einem HLB-Wert im Bereich von 12 - 18 enthalten ist. Derartige Emulsionen zeichnen sich durch eine besonders günstige Lager- und Temperaturstabilität aus.

Weitere bevorzugte erfindungsgemäß erhältliche Öl-in-Wasser-Emulsionen sind dadurch gekennzeichnet, dass das Gewichtsverhältnis von nichtionischen Emulgatoren mit einem HLB-Wert im Bereich von 3 - 6 und nichtionischen Emulgatoren mit einem HLB-Wert im Bereich von 12 - 18 von 0,9 bis 3, bevorzugt 1,3 -1,9 beträgt.

In der nachfolgenden Tabelle sind verschiedene Öl-in-Wasser-Emulgatoren und Wasser-in-ÖI-Emulgatoren und ihre HLB-Werte zusammengestellt. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. Die HLB-Werte können nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.
- HLB-Wert: Chemische Bezeichnung
- 1: Triglyceride gesättigter Fettsäuren Glyceryltrioleat
- 1,5: Ethylenglycoldistearat
- 1,6: Pur-Cellinöl
- 1,8: Sorbitantrioleat Glycerindioleat
- 2,1: Sorbitantristearat
- 2,4: Propylenglycollactostearat
- 2,7: Glycerinmonooleat Sorbitdioleat
- 2,8: Glycerinmonostearat Propylenglycolmono-/distearat, nicht selbstemulgierend
- 2,9: Ethylenglycolmonostearat
- 3,0: Decaglycerindecaoleat Decaglycerindecastearat Generol 122 (Rapeseed Sterols) Sucrosedistearat
- 3,1: Decaglycerindecaoleat Glycerylmonoricinoleat Pentaerythritylmonostearat Pentaerythritylsesquioleat
- 3,2: Ethylenglycolmonodistearat, nicht selbstemulgierend Glycolstearat
- 3,3: Glycerinmonolaurat
- 3,4: Propylenglycolmonostearat
- 3,5: Ethylenglycolmonostearat Pentaerythritylmonooleat Polyethylenglycol(100)monooleat
- 3,6: Glycerinmono-/dioleat, nicht selbstemulgierend Monoethoxylaurylether
- 3,7: Sorbitansesquioleat (Dehymuls SSO)
- 3,8: Glycerinmonodistearat, nicht selbstemulgierend Polyethylenglycol(100)monostearat Diglycerinsesquioleat N,N-Dimethylcaproamid Pentaerythritmonotallat Propylenglycolmonolaurat
- 4,0: Decaglycerinoctaoleat
- 4,3: Sorbitanmonooleat (Dehymuls SMO) Diethylenglycolmonostearat
- 4,4: 1.2-Propylenglycolmonodistearat, selbstemulgierend
- 4,5: Glycerinmonostearatpalmitat (90 %), nicht selbstemulgierend Propylenglycolmonolaurat
- 4,7: Sorbitanmonostearat (Dehymuls SMS) Diethylenglycolmonooleat
- 4,8: Pentaerythritmonolaurat
- 4,9: Polyoxyethylen(2)oleylalkohol (Polyoxyethylen(2)oleylether) Polyoxyethylen(2)stearylalkohol (Polyoxyethylen(2)stearylether)
- 5,0: Ethylenglycolmonodistearat Generol 122 E 5 (PEG-5 Soy Sterol) Polyethylenglycol(100)monoricinoleat Polyethylenglycol(200)distearat Polyglyceryl-3-isostearate (z. B. Isolan GI 34 von Tego)
- 5,9: Polyethylenglycol(200)dilaurat
- 6,0: Decaglycerintetraoleat Polyethylenglycol(100)monolaurat Polyethylenglycol(200)dioleat
- 6,1: Diethylenglycolmonolaurat (Diglycollaurat)
- 6,3: Polyethylenglycol(300)dilaurat
- 6,4: Glycerinmonoricinoleat Glycerinsorbitanmonolaurat
- 6,5: Diethylenglycolmonolaurat Natriumstearoyl-2-lactylat
- 6,7: Sorbitanmonopalmitat
- 6,8: Glycerinmonococoat Glycerinmonolaurat
- 7,0: Polyoxyethylen(2)C₁₀-C₁₄-fettalkoholether, Laureth-2 (Dehydol LS 2) Saccharosedistearat
- 7,2: Polyethylenglycol(400)dioleat Saccharosedioleat
- 7,4: Polyethylenglycol(100)monolaurat Saccharosedipalmitat
- 7,5: Saccharosedipalmitat
- 7,6: Glycerinsorbitanlaurat
- 7,8: Polyethylenglycol(400)distearat
- 7,9: Polyethylenglycol(200)monostearat Polyoxyethylen(3)tridecylalkohol
- 8-8,2: Polyethylenglycol(400)distearat
- 8,0: Polyoxyethylen(3)C₁₀-C₁₄-fettalkoholether, Laureth-3 (Dehydol LS 3) N.N-Dimethyllauramid Natriumlauroyllactylat, Natriumlauroyl-2-lactylat Polyethylenglycol(200)monooleat Polyethylenglycol(220)monotallat Polyethylenglycol(1500)dioleat Polyoxyethylen(4)oleylalkohol Polyoxyethylen(4)stearylcetylether
- 8,2: Triglycerinmonooleat
- 8,3: Diethylenglycolmonolaurat
- 8,4: Polyoxyethylen(4)cetylether Polyoxyethylenglycol(400)dioleat
- 8,5: Natriumcaproyllactylat Polyethylenglycol(200)monostearat Sorbitanmonooleat
- 8,6: Sorbitanmonolaurat (Dehymuls SML) Polyethylenglycol(200)monolaurat
- 8,8: Polyoxyethylen(4)myristylether Polyethylenglycol (400)dioleat
- 8,9: Nonylphenol, polyoxyethyliert mit 4 Mol EO
- 9,0: Oleth-5 (z. B. Eumulgin O 5)
- 9,2 - 9,7: Polyoxyethylen(4)laurylalkohol (je nach Handelsprodukt, z. B. Brij 30, Dehydol LS 4)
- 9,3: Polyoxyethylen(4)tridecylalkohol
- 9,6: Polyoxyethylen(4)sorbitanmonostearat
- 9,8: Polyethylenglycol (200)monolaurat
- 10-11: Polyethylenglycol(400)monooleat
- 10,0: Didodecyldimethylammoniumchlorid
- 10,0: Polyethylenglycol(200)monolaurat Polyethylenglycol(400)dilaurat Polyethylenglycol(600)dioleat Polyoxyethylen(4)sorbitanmonostearat Polyoxyethylen(5)sorbitanmonooleat
- 10,2: Polyoxyethylen(40)sorbitol hexaoleat
- 10,4 - 10,6: Polyoxyethylenglycol(600)distearat
- 10,5: Polyoxyethylen(20)sorbitantristearat
- 10,6: Saccharosemonostearat
- 10,7: Saccharosemonooleat
- 11 - 11,4: Polyethylenglycol(400)monooleat
- 11,0: Polyethylenglycol(350)monostearat Polyethylenglycol(400)monotallat Polyoxyethylenglycol(7)monostearat Polyoxyethylenglycol(8)monooleat Polyoxyethylen(20)sorbitantrioleat Polyoxyethylen(6)tridecylalkohol
- 11,1: Polyethylenglycol(400)monostearat
- 11,2: Polyoxyethylen(9)monostearat Saccharosemonooleat Saccharosemonostearat
- 11,4: Polyoxyethylen(50)sorbitol hexaoleat Saccharosemonotallat Saccharosestearatpalmitat
- 11,6: Polyoxyethylenglycol(400)monoricinoleat
- 11,7: Saccharosemonomyristat Saccharosemonopalmitat
- 12,0: PEG-10 Soy Sterol (z. B. Generol 122 E 10) Triethanolaminoleat
- 12,2-12,3: Nonylphenol, ethoxyliert mit 8 Mol EO
- 12,2: Saccharosemonomyristat
- 12,4: Saccharosemonolaurat Polyoxyethylen(10)oleylalkohol, Polyoxyethylen(10)oleylether Polyoxyethylen(10)stearylalkohol, Polyoxyethylen(10)stearylether
- 12,5: Polyoxyethylen(10)stearylcetylether
- 12,7: Polyoxyethylen(8)tridecylalkohol
- 12,8: Polyoxyethylenglycol(400)monolaurat Saccharosemonococoat
- 12,9: Polyoxyethylen(10)cetylether
- 13: Glycerinmonostearat, ethoxyliert (20 Mol EO)
- 13,0: Eumulgin O 10 (Polyoxyethylen(10)oleylether) Eumulgin 286 (Nonoxynol-10) Eumulgin B 1 (Ceteareth-12)
- 13,0: C12-Fettamine, ethoxyliert (5 Mol EO)
- 13,1: Nonylphenol, ethoxyliert (9,5 Mol EO)
- 13,2: Polyethylenglycol(600)monostearat Polyoxyethylen(16)tallöl
- 13,3: Polyoxyethylen(4)sorbitanmonolaurat
- 13,5: Nonylphenol, ethoxyliert (10,5 Mol EO) Polyethylenglycol(600)monooleat
- 13,7: Polyoxyethylen(10)tridecylalkohol Polyethylenglycol(660)monotallat Polyethylenglycol(1500)monostearat Polyoxyethylenglycol(1500)dioleat
- 13,9: Polyethylenglycol(400)monococoat Polyoxyethylen(9)monolaurat
- 14-16: Eumulgin HRE 40 (Ricinusöl, mit 40 EO ethoxyliert und hydriert)
- 14,0: Polyoxyethylen(12)laurylether Polyoxyethylen(12)tridecylalkohol
- 14,2: Polyoxyethylen(15)stearylalkohol
- 14,3: Polyoxyethylen(15)stearylcetylether
- 14,4: Gemisch aus C12-C15-Fettalkoholen mit 12 Mol EO
- 14,5: Polyoxyethylen(12)laurylalkohol
- 14,8: Polyoxyethylenglycol(600)monolaurat
- 14.9 - 15,2: Sorbitanmonostearat, mit 20 EO ethoxyliert (z. B. Eumulgin SMS 20)
- 15 - 15,9: Sorbitanmonooleat, mit 20 EO ethoxyliert (z. B. Eumulgin SMO 20)
- 15,0: PEG-20 Glyceryl stearate (z. B. Cutina E 24) PEG-40 Castor Oil (z. B. Eumulgin RO 40) Decylglucosid (Oramix NS 10) Dodecylglucosid (Plantaren APG 600) Dodecyltrimethylammoniumchlorid Nonylphenol, ethoxyliert mit 15 Mol EO Polyethylenglycol(1000)monostearat Polyoxyethylen(600)monooleat
- 15-17: Eumulgin HRE 60 (Ricinusöl, mit 60 EO ethoxyliert und hydriert)
- 15,3: C12-Fettamine, polyoxyethyliert mit 12 Mol EO Polyoxyethylen(20)oleylalkohol, Polyoxyethylen(20)oleylether
- 15,4: Polyoxyethylen(20)stearylcetylether (z. B. Eumulgin B 2 (Ceteareth-20))
- 15,5: Polyoxyethylen(20)stearylalkohol
- 15,6: Polyoxyethylenglycol(1000)monostearat Polyoxyethylen(20)sorbitanmonopalmitat
- 15,7: Polyoxyethylen(20)cetylether
- 15,9: Dinatriumtriethanolamindistearylheptaglycolethersulfosuccinat
- 16,0: Nonylphenol ethoxyliert mit 20 Mol EO Polyoxyethylen(25)propylenglycolstearat
- 16 - 16,8: Polyoxyethylen(30)monostearat
- 16,3-16,9: Polyoxyethylen(40)monostearat
- 16,5 - 16,7: Polyoxyethylen(20)sorbitanmonolaurat (z. B. Eumulgin SML 20)
- 16,6: Polyoxyethylen(20)sorbit
- 16,7: C18-Fettamine, polyoxyethyliert mit 5 Mol EO Polyoxyethylen(23)laurylalkohol
- 17,0: Ceteareth-30, z. B. Eumulgin B 3 Octylglucosid (Triton CG 110) Polyoxyethylen(30)glycerylmonolaurat
- 17,1: Nonylphenol, ethoxyliert mit 30 Mol EO
- 17,4: Polyoxyethylen(40)stearylalkohol

Weitere besonders bevorzugte erfindungsgemäß erhältliche Öl-in-Wasser-Emulsionen sind dadurch gekennzeichnet, dass Steareth-2, Steareth-21 und PPG-15-Stearylether enthalten sind. Derartige Emulsionen zeichnen sich durch eine besonders hohe Lager- und Temperaturstabilität aus und tragen gleichzeitig zu einem verbesserten, nicht-klebrigen Hautgefühl bei.

Außerordentlich bevorzugte erfindungsgemäß erhältliche Öl-in-Wasser-Emulsionen sind dadurch gekennzeichnet, dass neben Steareth-2, Steareth-21 und PPG-15-Stearylether Aluminiumstärkeoctenylsuccinat als Polysaccharid enthalten ist. Dieses Polysaccharid ist beispielsweise unter den Handelsnamen Dry Flo und Dry Flo Plus von National Starch erhältlich. Weitere außerordentlich bevorzugte erfindungsgemäß erhältliche Öl-in-Wasser-Emulsionen sind dadurch gekennzeichnet, dass neben Steareth-2, Steareth-21 und PPG-15-Stearylether mindestens ein Distärkephosphat enthalten ist. Dieses Polysaccharid ist beispielsweise unter dem Handelsnamen Maize PO 4 PH "B" von Agrana erhältlich. Derartige Emulsionen zeichnen sich durch eine besonders hohe Lager- und Temperaturstabilität, ein hervorragendes, nicht-klebriges Hautgefühl und optimale Trocknungseigenschaften auf der Haut aus.

### Wasser

Der Anteil des Wassers an den erfindungsgemäß erhältlichen Emulsionen beträgt mindestens 60 Gew.-%, bevorzugt 65 bis 90 Gew.-%, besonders bevorzugt 70 - 85 Gew.-%, außerordentlich bevorzugt 75 - 80 Gew.-%, jeweils bezogen auf die Gesamtzusammensetzung.

Weitere bevorzugte erfindungsgemäß erhältliche Öl-in-Wasser-Emulsionen sind dadurch gekennzeichnet, dass insgesamt maximal 3 Gew.-%, bevorzugt maximal 1 Gew.-% und besonders bevorzugt 0 Gew.-%, jeweils bezogen auf das Gewicht der gesamten Emulsion, an einwertigen C₁ - C₃-Alkanolen, wie Ethanol oder Isopropanol, enthalten ist. Die erfindungsgemäß erhältlichen Emulsionen können unter bestimmten Bedingungen durch einen Zusatz an Ethanol oder Isopropanol, insbesondere in höheren Mengen, beispielsweise 5 Gew.-% und mehr, in ihrer Lager- und/oder Temperaturstabilität destabilisiert werden.

Die erfindungsgemäß erhältlichen Öl-in-Wasser-Emulsionen wurden insbesondere für Röll-on-Produkte entwickelt, das heißt, für die Applikation mit einem Kugelapplikator oder Roll-on-Applikator. Für optimale Dosiereigenschaften darf die Emulsion weder zu niedrig-viskos noch zu hoch-viskos sein. Bevorzugte erfindungsgemäß erhältliche Öl-in-Wasser-Emulsionen sind daher durch eine Viskosität im Bereich von 1000 - 5000 mPas, bevorzugt 1500 - 4000 mPas und besonders bevorzugt 1700 - 2200 mPas gekennzeichnet. Diese Viskositätsangaben beziehen sich auf Messungen mit einem Brookfield-Viskosimeter, die 1 Tag nach Herstellung der Emulsion mit Spindel RV 4, bei einer Scherrate (Umdrehungszahl der Spindel) von 20 s⁻¹ ohne Helipath bei einer Umgebungstemperatur und einer Probentemperatur von jeweils 20 °C durchgeführt werden.

### Schweißhemmende Wirkstoffe

Bevorzugte erfindungsgemäß erhältliche Öl-in-Wasser-Emulsionen sind dadurch gekennzeichnet, dass der kosmetische Wirkstoff c) ausgewählt ist aus schweißhemmenden Wirkstoffen. Erfindungsgemäß bevorzugte schweißhemmende oder Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze, enthalten ist. Besonders bevorzugte Antitranspirant-Wirkstoffe sind ausgewählt aus den Aluminiumchlorhydraten, insbesondere den Aluminiumchlorhydraten mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorohydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEGdichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe werden erfindungsgemäß bevorzugt als wässrige Lösungen eingesetzt. Bei Verwendung von Zirconiumsalzen und Aluminium-Zirconium-Salzen ist zu beachten, dass die vorgefertigten wässrigen Wirkstofflösungen möglichst frisch zubereitet sind. Bei längerer Lagerzeit können die Zirconiumverbindungen zur Polymerisation neigen, was mit einem Aktivitätsverlust als auch mit einer Zunahme der Viskosität einhergeht.

Besonders bevorzugte erfindungsgemäß erhältliche schweißhemmende Emulsionen sind dadurch gekennzeichnet, dass der mindestens eine Antitranspirant-Wirkstoff in einer Menge von 1 - 38 Gew.-%, vorzugsweise 5 - 25 Gew.-% und insbesondere 10 - 20 Gew.-%, enthalten ist, jeweils bezogen auf das Gesamtgewicht der Aktivsubstanz in der Gesamtzusammensetzung. In einer besonders bevorzugten Ausführungsform enthält die Zusammensetzung ein adstringierendes Aluminiumsalz, insbesondere Aluminiumchlorohydrat, das beispielsweise in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.

Es kann erfindungsgemäß bevorzugt sein, die gesamte Wassermenge in mehreren Portionen zum Versuchsansatz zuzugeben und einzuhomogenisieren, insbesondere im Temperaturbereich von 70- 80 °C. Vorteilhaft ist eine Portionierung in fünf Fünftel oder sechs Sechstel der gesamten Wassermenge, wovon 2 oder 3 Portionen im höheren Temperaturbereich und 2 oder 3 Portionen im niedrigeren Temperaturbereich zugegeben werden.

### Desodorierende Wirkstoffe

Weitere bevorzugte erfindungsgemäß erhältliche Öl-in-Wasser-Emulsionen sind dadurch gekennzeichnet, dass der kosmetische Wirkstoff c) ausgewählt ist aus desodorierenden Wirkstoffen. Erfindungsgemäß bevorzugte desodorierende Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u.a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Zinklactat, Dinatriumdihydroxyethylsulfosuccinylundecylenat, Natriumbicarbonat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerin-ether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) als keimhemmende Wirkstoffe bevorzugt.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus Picea *spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.

Zu den Enzyminhibitoren gehören im Sinne der vorliegenden Erfindung Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. bevorzugt Trialkylcitronensäureester, insbesondere Triethylcitrat, oder Zinkglycinat. Weitere Inhibitorsubstanzen der für die Schweißzersetzung verantwortlichen Enzyme und Keime, beispielsweise Arylsulfatase, β-Glucuronidase, Aminoacylase, Esterasen, Lipasen und/oder Lipoxigenase, sind offenbart in WO 03/039505 A2, WO 01/99376 A2, EP 1430879 A2, EP 1428520 A2, EP 1738803 A1, EP 1576946 A1 und DE 10216368 A1.

Weitere erfindungsgemäß bevorzugte Emulsionen sind dadurch gekennzeichnet, dass mindestens ein desodorierender Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, jeweils bezogen auf das Gesamtgewicht der Aktivsubstanz in der Gesamtzusammensetzung, enthalten ist.

Weitere bevorzugte erfindungsgemäß erhältliche Emulsionen sind dadurch gekennzeichnet, dass mindestens ein kosmetischer Wirkstoff c), ausgewählt aus Monomeren, Oligomeren oder Polymeren von Aminosäuren, N-C₂-C₂₄-Acylaminosäuren und/oder den Estern und/oder den physiologisch verträglichen Salzen dieser Substanzen, enthalten ist. Viele dieser Wirkstoffe werden als Anti-ageing-Wirkstoffe eingesetzt und/oder wirken günstig auf den Feuchtigkeitshaushalt der Haut ein und/oder haben eine hautberuhigende Wirkung.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die Aminosäuren, insbesondere die genannten bevorzugten Aminosäuren, an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Aminosäuren oder Aminosäurederivate sind ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-ß (*tissue growth factor*), der die Synthese von Collagen in dermalen Fibroblasten induziert, freizusetzen. Derartige Aminosäureoligomere werden bevorzugt als Wirkstoffe gegen die Hautalterung verwendet.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-ß-Ala-His, N-Acetyl-Tyr-Arg-hexyldecylester (z. B. Calmosensine von Sederma), Camosin (ß-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Om, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapeptide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Om, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.

Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß bevorzugt sind auch kationisierte Proteinhydrolysate. Besonders bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quatemierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association, Washington, DC) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/ Myristyl Ether HCl. Außerordentlich bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.

Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A*. *nidulans* wird in technisch relevanten Mengen mittlerweile aus E. coli gewonnen. Photolyase ist zur Aktivierung auf Licht angewiesen.

Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

Bevorzugte erfindungsgemäße Emulsionen sind dadurch gekennzeichnet, dass sie mindestens einen der Rohstoffe Photosome™ oder Ultrasome™ in einer Gesamtmenge von 0,1 - 10 Gew.-%, bevorzugt 0,5 - 5,0 Gew.-% und besonders bevorzugt 1,0 - 4,0 Gew.-%, jeweils bezogen auf die gesamte Emulsion, enthalten.

Bevorzugte erfindungsgemäße Emulsionen sind dadurch gekennzeichnet, dass sie mindestens ein Monomer, Oligomer oder Polymer von Aminosäuren, N-C₂-C₂₄-Acylaminosäuren und/oder den Estern und/oder den physiologisch verträglichen Salzen dieser Substanzen in einer Gesamtmenge von 0,01 - 10 Gew.-%, bevorzugt 0,1 - 5 Gew.-% und besonders bevorzugt 0,1 - 3 Gew.-%, jeweils bezogen auf die gesamte Emulsion, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Salzen dieser Substanzen in geträgerter Form vor, insbesondere aufgetragen auf feinteiligen, pulverförmigen Substraten wie Kieselgel, insbesondere Aerosil-Typen, Talkum, modifizierten Stärken und Stärkederivaten, kristalliner Cellulose, Cellulosepulvern, Lactoglobulinderivaten, Microsponges, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Erfindungsgemäß besonders bevorzugte kosmetische Emulsionen sind dadurch gekennzeichnet, dass sie mindestens einen kosmetischen Wirkstoff c), der ausgewählt ist aus Monomeren, Oligomeren und Polymeren von Aminosäuren, N-C₂-C₂₄-Acylaminosauren und/oder den Estern und/oder den physiologisch verträglichen Salzen dieser Substanzen, in einer Gesamtmenge von 0.000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, jeweils bezogen auf den Gehalt an Aktivsubstanz in der gesamten Emulsion, enthalten.

In einer weiteren bevorzugten Ausführungsform enthalten die erfindungsgemäß erhältlichen Öl-in-Wasser-Emulsionen als kosmetischen Wirkstoff c) mindestens ein DNA-Oligonucleotid oder ein RNA-Oligonucleotid. Diesen Komponenten werden positive Effekte insbesondere bei der Anti-Falten- und der Anti-Ageing-Behandlung zugeordnet

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).

Ein erfindungsgemäß besonders bevorzugtes Oligonucleotid ist das Thymidin-Dinucleotid.

Bevorzugte erfindungsgemäß erhältliche Öl-in-Wasser-Emulsionen sind dadurch gekennzeichnet, dass sie mindestens ein DNA-Oligonucleotid oder RNA-Oligonucleotid in einer Gesamtmenge von 0,0001 - 5 Gew.-%, bevorzugt 0,001 - 1,0 Gew.-% und besonders bevorzugt 0,01 - 0,5 Gew.-%, bezogen auf die gesamte Emulsion, enthalten.

In einer weiteren bevorzugten Ausführungsform enthalten die erfindungsgemäß erhältlichen Öl-in-Wasser-Emulsionen als kosmetischen Wirkstoff c) mindestens eine natürliche Betainverbindung. Diese Komponenten haben positive Effekte insbesondere bei der hautbefeuchtenden Behandlung. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺CH₂-COO⁻) und Camitin (Me₃N⁺-CH₂-CHOH-CH₂-COO⁻), jeweils mit Me = Methyl.

Bevorzugte erfindungsgemäß erhältliche Öl-in-Wasser-Emulsionen sind dadurch gekennzeichnet, dass sie mindestens eine natürliche Betainverbindüng in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Emulsion, enthalten.

In einer weiteren bevorzugten Ausführungsform enthalten die erfindungsgemäß erhältlichen Öl-in-Wasser-Emulsionen als kosmetischen Wirkstoff c) mindestens ein Vitamin, Provitamin oder eine als Vitaminvorstufe bezeichnete Verbindung aus den Vitamingruppen A, B, C, E, H und K und den Estern der vorgenannten Substanzen. Diesen Komponenten werden positive Effekte insbesondere bei der Anti-Falten- und der Anti-Ageing-Behandlung, aber auch bei der hautbefeuchtenden, aufhellenden, sebumregulierenden und hautberuhigenden Behandlung zugeordnet.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfingdungsgemäß beispielsweise Vitamin A-Säure und deren Ester, Vitamin A-Aldehyd und Vitamin Ar-Alkohol sowie dessen Ester, wie Retinylpalmitat und Retinylacetat in Betracht. Die erfindungsgemäß erhältlichen Emulsionen enthalten die mindestens eine Vitamin A-Komponente bevorzugt in einer Gesamtmenge von 0,05 - 1 Gew.-%, bezogen auf die gesamte Emulsion.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3H,10H)-dion. Bevorzugt werden Riboflavin oder seine Derivate in einer Gesamtmenge von 0,05 bis 1 Gew.-%, bezogen auf die gesamte Zusammensetzung, eingesetzt.
- Vitamin Bs. Unter dieser Bezeichnung werden die Verbindungen Nicotinsäure und Nicotinsäureamid (Niacinamid) geführt. Erfindungsgemäß bevorzugt Ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin Bs (Pantothensäure und Panthenol). Bevorzugt wird Panthenol eingesetzt. Erfindungsgemaß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-I) eingesetzt werden.

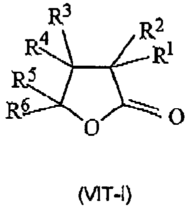

Bevorzugt sind die 2-Furanon-Derivate, in denen die Substituenten R¹ bis R⁶ unabhängig voneinander ein Wasserstoffatom, einen Hydroxylrest. einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trinydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Akirich) und 2,5-Dihydro-rmethoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäß erhältlichen Zusammensetzungen bevorzugt In einer Gesamtmenge von 0,05 bis 5 Gew.-%, besonders bevorzugt 0,1 bis 3 Gew.-%, außerordentlich bevorzugt 0,5 bis 2 Gew.-%, jeweils bezogen auf die gesamte Zusammensetzung, enthalten.
- Vitamin B₆; wobei man hierunter keine einheitliche Substanz, sondern die unter den Trivialnamen Pyridoxin, Pyridoxamin und Pyridoxal bekannten Derivate des 5-Hydroxy-methyl-2-methylpyridin-3-ols versteht. Vitamin B₆ ist in den erfindungsgemäß erhältlichen Zusammensetzungen bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*,6a*R*)-2-Oxohexahydrothienol[3,4-*d*]-imidazol-4-valeriansäure. Biotin ist in den erfindungsgemäß erhältlichen Zusammensetzungen bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-{2-Amino-3,4-dihydro-4-oxo-6-pteridinylrnethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Amino-benzoesäure und L-Glutaminsäure verbundenen Pteridin-Ring enthält. Folsäure ist ein Wachstumsfaktor für verschiedene Mikroorganismen und eine Verbindung mitVitamincharakter, die in der Natur meist als Polyglutamat und in reduzierter Form (7,8-Dihydrofolsäure, H₂Folat, DHF; Tetrahydrofolsäure, H₄Folat, THF; 5-Methyl-Tetr-ahydrofolsäure, CH₃-H₄Folat, MeTHF) vorkommt. Besonders bevorzugt erfindungsgemäß erhältliche Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfingdungsgemäß besonders bevorzugt. Besonders bevorzugt erfindungsgemäß erhältliche Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin C-Gruppe zählen Vitamin C (Ascorbinsäure) und seine Derivate, insbesondere die Ester der Ascorbinsäure mit organischen und anorganischen Säuren und deren Salze, sowie die Acetale mit Glucose oder anderen Zuckern, insbesondere Ascorbylglucosid. Vitamin C und/oder mindestens eines seiner Derivate wird bevorzugt in einer Gesamtmenge von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung mindestens eines Mitglieds der Vitamin C - Gruppe in Kombination mit Tocopherolen und/oder anderen Mitgliedern der Vitamin E - Gruppe kann ebenfalls bevorzugt sein.

Zur Vitamin E-Gruppe zählen Tocopherol, Insbesondere α-Tocopherol, und seine Derivate. Bevorzugte Derivate sind insbesondere die Ester, wie Tocopherylacetat, -nicotinat -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in einer Gesamtmenge von 0,05 - 1 Gew.-%, bezogen auf die gesamte Zusammensetzung, enthalten.

Vitamin H Ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Famochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Emulsion, enthalten. Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugte Wirkstoffe c).

in einer weiteren bevorzugten Ausführungsform enthalten die erfindungsgemäß erhältlichen Öl-in-Wasser-Emulsionen als kosmetischen Wirkstoff c) mindestens ein Vitamin, Provitamin oder eine als Vitaminvorstufe bezeichnete Verbindung aus den Vitamingruppen A, B, C, E, H und K und den Estern der vorgenannten Substanzen in einer Gesamtmenge von 0,1 bis 5 Gew.-%, vorzugsweise von 0,25 bis 4 Gew.% und insbesondere von 0,5 bis 2,5 Gew.-%, Jeweils bezogen auf die gesamte Emulsion.

In einer weiteren bevorzugten Ausführungsform enthalten die erfindungsgemäß erhältlichen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton.

In einer weiteren bevorzugten Ausführungsform enthalten die erfindungsgemäß erhältlichen Ol-in-Wasser-Emulsionen als kosmetischen Wirkstoff c) mindestens eine α-Hydroxycarbonsäure, α-Ketocarbonsäure oder β-Hydroxycarbonsäure oder deren Ester-, Lacton- oder Salzform. Diesen Komponenten werden positive Effekte insbesondere bei der Anti-Falten- und der Anti-Ageing-Behandlung, aber auch bei der hautbefeuchtenden bzw. feuchtigkeitsspendenden, aufhellenden, sebumregulierenden und Anti-Akne-Behandlung zugeordnet. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansaure, 2-HydroxyheptansAure, 2-Hydroxyoctansäure, 2-Hydroxydecansäure, 2-Hydroxydodecansäure, 2-Hydroxytetradecansäure, 2-Hydroxyhexadecansäure, 2-Hydroxyocta-decansäure, Mandelsäure. 4-Hydroxymandelsäure, Äpfelsäure, Erythrarsäure. Threarsäure, Glucarsäure, Galactarsäure, Mannarsäure, 2-Hydroxy-2-methylbernsteinsäure, Gluconsäure, Gularsäure, Brenztraubensäure, Glucuronsäure und Galacturonsaure. Besonders bevorzugte α-Hydroxycarbonsäuren sind Milchsäure, Citronensäure, Glycolsäure und Gluconsäure. Eine besonders bevorzugte β-Nydroxycarbonsäure ist Salicylsäure. Die Ester der genannten Säuren sind bevorzugt ausgewählt aus den Methyl-, Ethyl-, Propyl-, Isopropyl-, Butyl-, Amyl-, Pentyl-, Hexyl-, 2-methylhexyl-, Octyl-, Decyl-, Dodecyl- und Hexadecylestern. Besonders bevorzugte erfindungsgemäße Emulsionen sind dadurch gekennzeichnet, dass mindestens eine α-Hydroxycarbonsäure, α-Ketocarbonsäure und/ oder β-Hydroxycarbonsäure und/oder mindestens ein Derivat hiervon in einer Gesamtmenge von 0,1 - 10 Gew.-%, bevorzugt 0,5 - 5 Gew.-%, jeweils bezogen auf die gesamte Emulsion, enthalten ist.

In einer weiteren bevorzugten Ausfohrungsform enthalten die erfindungsgemäß erhältlichen Öl-in-Wasser-Emulsionen als kosmetischen Wirkstoff c) mindestens ein Flavonoid und/oder mindestens einen Flavonoid-reichen Pflanzenextrakt. Diesen Komponenten werden positive Effekte insbesondere bei der Anti-Falten- und der Anti-Ageing-Behandlung, aber auch bei der hautbefeuchtenden bzw. feuchtigkeitsspendenden, aufhellenden, sebumregulierenden und Anti-Akne-Behandlung zugeordnet.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Nanngenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-mamhoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl}-β-D-glucopyranosid)), Diosmin (3',4',7-Trihydroxy-5-methoxyflavanon-7-rhamno-glucosid), Eriodictin und Apigenin-7-glucosid (4',5,7-Trihydroxyflavon-7-glucosid). Erfingdungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Besonders bevorzugte erfindungsgemäß erhältliche Emulsionen sind dadurch gekennzeichnet, dass mindestens ein Flavonoid und/oder mindestens einen Flavonoid-reicher Pflanzenextrakt in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten Emulsion, enthalten ist.

In einer weiteren bevorzugten Ausführungsform enthalten die erfindungsgemäß erhältlichen Öl-in-Wasser-Emulsionen als kosmetischen Wirkstoff c) mindestens ein Isoflavonoid oder mindestens einen Isoflavonoid-reichen Pflanzenextrakt. Zu den Isoflavonoiden werden an dieser Stelle die Isöflavone und die Isoflavon-Glycoside gezählt. Diesen Komponenten werden positive Effekte Insbesondere bei der Anti-Falten- und der Anti-Ageing-Behandlung zugeordnet

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daldzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galaeturonsäure, D-Xylose, D-Apiose, L-Rhamnose, L-Arabinose und Rutinose in Betracht Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkemextrakt, insbesondere das Handelsprodukt Ederline von Seporga. Ederline enthält Phytohormone, Isaflavonoide, Phytosterole, Triterpenoide, Tocopherole und natürliche Wachse. Besonders bevorzugte erfindungsgemäß erhältliche Emulsionen sind dadurch gekennzeichnet, dass als kosmetischer Wirkstoff c) mindestens ein Isoflavonoid und/oder mindestens ein Isoflavonoid-reicher Pflanzenextrakt in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten Zusammensetzung, enthalten ist.

In einer weiteren bevorzugten Ausführungsform enthalten die erfindungsgemäß erhältlichen Öl-n-Wasser-Emulsionen als kosmetischen Wirkstoff c) mindestens ein Polyphenol und/oder mindestens einen Polyphenol-reichen Pflanzenextrakt. Diesen Komponenten werden positive Effekte insbesondere bei der Anti-Falten-, der Anti-Ageing-Behandlung und der sebumregulierenden Hautbehandlung zugeordnet.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussaure-Derivate auf. Bevorzugte Polyphenole sind Flavone. Gatechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu. Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda. Besonders bevorzugte erfindungsgemäß erhältliche Emulsionen sind dadurch gekennzeichnet, dass mindestens ein Polyphenol und/oder mindestens ein Polyphenol-reicher Pflanzenextrakt in einer Gesamtmenge von 0,001 bis 10 Gew.-%, bevorzugt 0,005 bis 5 Gew.-% und besonders bevorzugt 0,01 bis 3 Gew.-%, jeweils bezogen auf die Polyphenolaktivsubstanz in der gesamten Emulsion, enthalten ist

In einer weiteren bevorzugten Ausführungsform enthalten die erfindungsgemäß erhältlichen Öl-in-Wasser-Emulsionen als kosmetischen Wirkstoff c) mindestens ein Ubichinon und/oder mindestens ein Ubichinol und/oder mindestens ein Derivat dieser Substanzen. Diesen Komponenten werden positive Effekte insbesondere bei der Anti-Falten- und der Anti-Ageing-Behandlung zugeordnet. Ubichinole sind die reduzierte Form der Ubichinone. Die erfindungsgemäß bevorzugten Ubichinone weisen die Formel (UBI-1) auf: 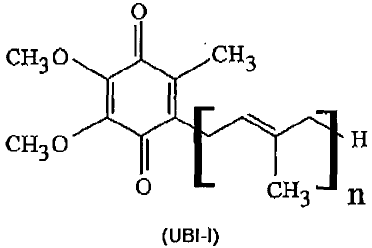 mit n = 6, 7, 8, 9 oder 10.

Besonders bevorzugt ist das Ubichinon der Formel (UBI-I) mit n = 10, auch bekannt als Coenzym Q10.

Besonders bevorzugte erfindungsgemäß erhältliche Emulsionen sind dadurch gekennzeichnet, dass mindestens ein Ubichinon und/oder mindestens ein Ubichinol und/oder mindestens ein Derivat dieser Substanzen in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Emulsion, enthalten ist.

In einer weiteren bevorzugten Ausführungsform enthalten die erfindungsgemäß erhältlichen Öl-In-Wasser-Emulsionen als kosmetischen Wirkstoff c) Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Diesen Komponenten werden positive Effekte insbesondere bei der hautberuhigenden Behandlung zugeordnet.

Besonders bevorzugte erfindungsgemäß erhältliche Emulsionen sind dadurch gekennzeichnet, dass Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Emulsion, enthalten ist.

In einer weiteren bevorzugten Ausführungsform enthalten die erfindungsgemäß erhältlichen Öl-in-Wasser-Emulsionen als kosmetischen Wirkstoff c) Ectoin. Dieser Komponente werden positive Effekte insbesondere bei der hautbefeuchtenden bzw. feuchtigkeitsspendenden Behandlung zugeordnet. Ectoin ist der Trivialname für 2-Methyl-1,4,5,6-tetrahydropyrimidin-4-carboxylat Besonders bevorzugte erfindungsgemäß erhältliche Emulsionen sind dadurch gekennzeichnet, dass Ectoin in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten ist.

In einer weiteren bevorzugten Ausführungsform enthalten die erfindungsgemäß erhältlichen Öl-in-Wasser-Emulsionen als kosmetischen Wirkstoff c) mindestens ein Repellent, das heißt, einen Wirkstoff zur Insektenabwehr.

Von den heute in Insektenabwehrmitteln ca. 15 häufig eingesetzten Wirkstoffen wird das N,N-Diethyl-3-methylbenzamid (DEET) als bestes Allround-Repellent bezeichnet Es wirkt abwehrend gegen Stechmücken, Bremsen, Sandfliegen, Zecken, Stechfliegen, Milben, Flöhe und Wanzen, wobei die Wirkungsdauer - wie bei allen Repellent-Wirkstoffen - unterschiedlich lang gegenüber den verschiedenen Spezies ist Handelsübliche DEET-Präparate beispielsweise sind ca. 6 bis 8 Stunden gegen Mücken wirksam, jedoch nur ca. 2 bis 4 Stunden gegen Zecken. Ein weiterer gebräuchlicher Repellent-Wirkstoff ist der 3-(N-n-Butyl-N-acetyl-amino)propionsäureethylester (auch als Repellent 3535 bezeichnet). Repellent 3535 ist gegen Stechmücken (Aedes aegypti, Anopheles albimanus), Tsetsefliegen (Glossinae) und Bremsen (Tabanidae) wirksam. Ferner gebräuchlich ist Dimethylphthalat (Palatinol M, DMP), das gegen Stechmücken (insbesondere Aedes- und Anopheles-Arten), Läuse, Zecken und Milben wirksam ist, allerdings vorwiegend in Kombination mit weiteren Repellent-Wirkstoffen eingesetzt wird.

In einer weiteren bevorzugten Ausführungsform enthalten die erfindungsgemäß erhältiichen Öl-in-Wasser-Emulsionen als kosmetischen Wirkstoff c) mindestens eine anorganische und/oder mindestens eine organische UV-Filtersubstanz.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestem und -carbonsäureamiden, Ketotricyclo(5.2.1,0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäß erhältlichen kosmetischen Zubereitungen vor. Es kann ggf. besonders bevorzugt sein, wenn die Benzoxazol-Derivate in pigmentarer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamlno)-benzoesaure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzyl-ester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamlno-2'-hydröxybenzoyl)-benzoesäurehexylester (auch: Aminobenzophenon, unter der Bezeichnung Uvinul A Plus bei der Firma BASF erhältlich). 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der INCI-Bezelchnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-{4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvi-nul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma). die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl) benzotriazol (CAS-Nr. 2440-22-4), 2-(2H-benzotrlazol-2-yl)-4-methyl-5-[2-methyl-3-[1,3,3,3-tetramethyl-9-((trimethylsilyl)oxy]disiloxanyl)propyl]-phenol (CAS-Nr.: 155633-54-8) mit der INCI-Bezeichnung Dro-metrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2n4-Bis-{[4-(2-ethyl hexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[14-(2-methy)propenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-{4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammo-nium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsaure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäß erhältlichen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus. 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat In diesen Kombinationen liegt das Gewichtsverhältnis von UV-B-Filter zu 1-(4-tert-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid. Silicate (Talk) und Bariumsulfat. Die Artikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärisch Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titan-dioxid und Zinkoxid.

Bevorzugte erfindungsgemäß erhältliche Emulsionen sind dadurch gekennzeichnet, dass sie mindestens eine organische UV-Filtersubsfanz in einer Gesamtmenge von 0,1 - 30 Gew.-%, bevorzugt 0,5 - 20 Gew.-%, besonders bevorzugt 1,0 - 10 Gew.-% und außerordentlich bevorzugt 2 oder 3 - 7 Gew.-%, jeweils bezogen auf die gesamte Zusammensetzung, enthalten.

Bevorzugte erfindungsgemaß erhältliche Zusammensetzungen sind dadurch gekennzeichnet, dass sie mindestens eine anorganische UV-Filtersubstanz in einer Gesamtmenge von 0,1 - 15 Gew.-%, bevorzugt 0,5 - 10 Gew.-%, besonders bevorzugt 1 - 5 Gew.-% und außerordentlich bevorzugt 2 - 4 Gew.-%, jeweils bezogen auf die gesamte Zusammensetzung, enthalten.

In einer weiteren bevorzugten Ausführungsform enthalten die erfindungsgemäß erhältlichen Öl-in-Wasser-Emulsionen als kosmetischen Wirkstoff c) mindestens einen selbstbräunenden Wirkstoff. Erfindungsgemäß bevorzugte selbstbräunende Wirkstoffe sind ausgewählt aus Dihydroxyaceton, Erythrulose und 5,6-Dihydroxyindolin sowie Mischungen dieser Komponenten, insbesondere Mischungen von Dihydroxyaceton und Erythrulose.

Bevorzugte erfindungsgemäß erhältlichen Emulsionen sind dadurch gekennzeichnet, dass sie mindestens einen selbstbräunenden Wirkstoff in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Emulsion, enthalten.

In einer weiteren bevorzugten Ausführungsform enthalten die erfindungsgemäß erhältlichen Öl-in-Wasser-Emulsionen als kosmetischen Wirkstoff c) mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsaure und/ oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat. Ascorbylmonooctanoat, Ascorbyldioctanoat, Ascorbylmonoisostearat und Ascorbyldiisostearat Die erfingdungsgemäß außerordentlich bevorzugten Ascorbinsäurederivate sind Natriumascorbylphosphat und Magnesiumascorbylphosphat.

Bevorzugte erfindungsgemäß erhältliche Emulsionen sind dadurch gekennzeichnet, dass sie mindestens einen hautaufhellenden Wirkstoff in einer Gesamtmenge von 0,05 bis 5 Gew.-%. bevorzugt 0,1 - 2 Gew-%, jeweils bezogen auf die gesamte Emulsion, enthalten.

In einer weiteren bevorzugten Ausführungsform enthalten die erfindungsgemäß erhältliche Öl-in-Wasser-Emulsionen als kosmetischen Wirkstoff c) mindestens einen hautberuhigenden Wirkstoff. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanlrea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vncience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobemsteinsäure (= Asparaginsäure), Aminoglutarsäure und Amlnoadipinsäure sowie deren physiologisch vertraglichen Salzen wie Kaliumaspartat und Magnesiumaspartat, sowie beliebigen Mischungen dieser Substanzen.

Weitere bevorzugte erfindungsgemäß erhältliche Emulsionen sind dadurch gekennzeichnet, dass sie mindestens einen hautberuhigenden Werkstoff in einer Gesamtmenge von 0,001 bis 5 Gew.-%, bevorzugt 0,01 bis 2 Gew.-% und besonders bevorzugt 0,1 bis 1 Gew.-%, jeweils bezogen auf die gesamte Emulsion, enthalten.

In einer weiteren bevorzugten Ausführungsform enthalten die erfindungsgemäß erhältlichen Öl-in-Wasser-Emulsionen als kosmetischen Wirkstoff c) mindestens einen feuchtigkeitsspendenden Wirkstoff. Erfindungsgemäß bevorzugte feuchtigkeitsspendende Wirkstoffe sind ausgewählt aus Desoxyzuckem, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabla, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N'-Bis(2-hydroxyethyl)hamstoff (z. B. erhältlich unter dem Handelsnamen Hydrovance), Betain (MeaN⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen.

Bevorzugte erfindungsgemäß erhältliche Emulsionen sind dadurch gekennzeichnet, dass sie mindestens einen feuchtigkeitsspendenden Wirkstoff in einer Gesamtmenge von 0,001 bis 10 Gew.-%, bevorzugt 0,1 bis 5 Gew.-% und besonders bevorzugt 1 bis 3 Gew.-%, jeweils bezogen auf die gesamte Emulsion, enthalten.

In einer weiteren bevorzugten Ausuhrungsform enthalten die erfindungsgemäß erhältlichen Öl-in-Wasser-Emulsionen als kosmetischen Wirkstoff c) mindestens einen sebumregulierenden Wirkstoff. Erfindungsgemäß bevorzugte sebumregulierende Wirkstoffe sind ausgewählt aus Azelainsäure, Azelainsäurederivaten, Insbesondere dem Azelainsaurederivat Potassium Azeloyl Diglycinate, das zum Beispiel als Handelsprodukt Azeloglicina von Sinerga erhältlich ist, Sebacinsäure, 10-Hydroxydecansaure, 1,10-Decandiol, Mischungen aus Sebacinsäure, 10-Hydroxydecansäure und 1,10-Decandiol, wie sie zum Beispiel als Handelsprodukt Acnacidol PG von Vincience erhältlich sind, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsaure(salzen) und/oder Gallotanninen und Naringin, wie sie zum Beispiel als Handelsprodukt BiSCos Glynarin PF von der Firma Biesterfeld erhältlich sind, weiterhin aus Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Serobiologiques), Hefeproteinhydrolysaten, wie sie z.B. in den Handelsprodukten der Asebiol^{®}-Serie von Laboratoires Sérobiologiques erhältlich sind, insbesondere Asebiol^{®} LS 2539 BT 2 (INCI: Aqua, Hydrolyzed Yeast Protein, Pyridoxine, Niacinamide, Glycerin, Panthenol, Allantoin; Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol). Bevorzugte erfindungsgemäß erhältliche Emulsionen sind dadurch gekennzeichnet, dass sie mindestens einen sebumregulierenden Wirkstoff in einer Gesamtmenge von 0,0001 bis 5 Gew.-%, bevorzugt 0,001 bis 2 Gew.-%, besonders bevorzugt 0,01 bis 1 Gew.-% und Außerordentlich bevorzugt 0,1 - 0,5 Gew.-%, jeweils bezogen auf den Aktivsubstanzgehalt in der gesamten erfindungsgemäß erhältlichen Emulsion, enthalten.

Die erfindungsgemäß erhältlichen Öl-in-Wasser-Emulsionen sind dadurch gekennzeichnet, dass die Öl- oder Fettphase mindestens einen Duftstoff umfasst.

Als Duftstoffkomponente können Parfüme, Parfümöle oder Parfümölbestandteile eingesetzt werden, Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat. Melusat und Jasmecyclat, Zu den Ethern zählen beispielsweise Benzylethylether und Ambroxan, zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zuganglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Laudanumöl sowie Orangenblutenöl, Neroliöl, Orangenschalenöl und Sandelhotzöl.

Um wahrnehmbar zu sein, muss ein Riechstoff flüchtig sein, wobei neben der Natur der funktionenlen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrucke in "Kopfnote° (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamotteöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefemnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl. Olibanumöl, Orangenöl, Origanumöl, Palmarosaöl, Patschullöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Stemanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylalkohol, n-Octylaldehyd, p-Oxy-Acetophenon, Pentadekanolid, β-Phenylethylalkohol, Phenylacetaldehyd-Dimethylacetal, Phenylessigsäure, Pulegon, Safrol, Salicylsaureisoamylester, Salicylsauremethylester, Salicylsäurehexylester, Salicylsäurecyclohexylester, Santalol, Skatol, Terpineol, Thymen, Thymol, r-Undelacton, Vanilin, Veratrumaldehyd, Zimtaldehyd, Zimatalkohol, Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butadion, Citral, Citronellal, Limonen, Linalool, Linaylacetat und -propionat, Menthol, Menthon, Methyl-n-heptenon, Phellandren, Phenylacetaldehyd und Terpinylacetat

Besonders bevorzugte erfindungsgemäß erhältliche kosmetische Emulsionen sind dadurch gekennzeichnet, dass mindestens eine Duftstoffkomponente in einer Gesamtmenge von 0,00001 bis 4 Gew.-%, bevorzugt 0,5 - 2 Gew.-%, besonders bevorzugt 1 - 1,5 Gew.-%, jeweils bezogen auf die Gesamtzusammensetzung, enthalten ist

Als optionale Ölkomponenten für die erfindungsgemaß erhältlichen O/W-Emulsionen eignen sich bevorzugt alle wasserunlöslichen, hautverträglichen Öle und Fettstoffe und deren Gemische mit festen Paraffinen und Wachsen, sofern sie von den Ölkomponente i, ii und iii verschieden sind. Der Schmelzpunkt der gegebenenfalls verwendeten Gemische mit festen Paraffinen oder Wachsen sollte Jedoch möglichst unterhalb der Phaseninversionstemperatur der Emulsion, bevorzugt unterhalb von 40 °C liegen. Außerdem sollte die Ölphase nicht zu unpolar sein.

Geeignete Ölkomponenten sind die Ester von gesättigten und ungesättigten, linearen und verzweigten C₃-C₂₂-Fettsäuren und den Dimeren ungesättigter C₁₂-C₂₂-Fettsäuren (Dimerfettsäuren) mit a) einwertigen linearen, verzweigten und cyclischen C₂-C₁₈-Alkanolen, b) mehrwertigen linearen und verzweigten C₂-C₆-Alkanolen, c) Polyglycerinen der Formel CH₂OH-CHOH-CH₂[-O-CH₂-CHOH-CH₂]ₙO-CH₂-CHOH-CH₂OH mit n = 0 - 8, und d) den Dicarbonsäureestern von linearen und verzweigten C₂-C₁₀-Alkanolen, den Benzoesäureestern von linearen und verzweigten C₁₀-C₂₀-Alkanolen, den C₁₂-C₂₂-Alkanolestern ein- und mehrwertiger C₂-C₇-Hydroxycarbonsäuren, die aromatisch sein können, sowie Mischungen dieser Komponenten.

Beispiele für erfindungsgemäß besonders bevorzugte Fettsäureester einwertiger linearer oder verzweigter C₂-C₁₈-Alkanole (Typ a) sind Octylethylhexanoat, Isopropylpalmitat, Cetyloleat, 2-Hexyldecyllaurat (im Gemisch mit 2-Hexyldecanol als Handelsprodukt Cetiol® PGL erhältlich), Hexyllaurat, Isopropylmyristat, ein Gemisch der Ester von Caprylsäure und Caprinsäure mit C₁₂-C₁₈-Fettalkoholen, das als Handelsprodukt Cetiol^{®} LC (Cognis) erhältlich ist, 2-Ethylhexylcaprylat, C₁₂-C₁₅-Alkyloctanoat, Isodecylneopentanoat, Isononylisononanoat, 2-Ethylhexylpalmitat (z. B. Cegesoft^{®} C 24 von Cognis), 2-Ethylhexylstearat, Cetearylisononanoat (Mischung aus Cetylisononanoat und Stearylisononanoat, z. B. Cetioi^{®} SN (Cognis)), Cetearyloctanoat, Ethylstearat, Isopropylstearat, Butyistearat und Myristylpropionat, außerdem natürlich vorkommende Esteröle wie z. B. Jojobaöl. Weiterhin geeignet sind die Monoester des Isosorbid (1,4:3,6-Dianhydro-D-glucit) mit C₂-C₂₄-Fettsäuren, z. B. das Isosorbidmonolaurat.

Beispiele für erfindungsgemäß besonders bevorzugte C₈-C₂₂-Fettsäureester mehrwertiger C₂-C₆-Alkanole (Typ b) sind als pflanzliche und tierische Öle vorkommende Fettsäuretriglycerid-Öle, z. B. Sonnenblumenöl, Sojaöl, Sesamöl, Haselnußöi, Olivenöl, Mandelöl, Rapsöl, Nachtkerzenöl (evening primrose oil), Distelöl (Safloröl), Avocadoöl sowie die flüssigen Anteile des Kokosöls oder des Rindertalgs, Erfindungsgemäß außerordentlich gut geeignet sind weiterhin synthetische Fettsäuretriglycerid-Öle. Besonders bevorzugt sind die Triglyceride von gesättigten C₈-C₁₀-Fettsäuren, wobei die Fettsäuren sowohl unverzweigt, wie z. B. bei den Handelsprodukten Myritol^{®} 318 und Myritol^{®} 331 (Cognis) oder Miglyol^{®} 812 (Hüls), als auch verzweigt sein können, wie z. B. bei den Handelsprodukten Estol^{®} GTEH 3609 (Uniqema) oder Myritol^{®} GTEH (Cognis).

Weitere bevorzugte Ester dieses Typs sind die Fettsäureester des Ethylenglycols, z. B. Ethylenglycoldioleat, Ethylenglycoldiisotridecanoat, des Butandiols, z. B. Butandioldiisostearat, des Neopentylglycols, z. B. Neopentylglycoldiheptanoat oder Neopentylglycoldicaprylat, des Trimethylolpropans und des Pentaerythrits. Beispiele für erfindungsgemäß bevorzugte Ölkomponenten sind die 1,2-Propylenglycoldiester von gesättigten, unverzweigten C₈-C₁₀-Fettsäuren, besonders bevorzugt die Handelsprodukte Myritol^{®} PC und Miglyol^{®} 840.

Beispiele für erfindungsgemäß besonders bevorzugte Dicarbonsäureester (Typ d) sind Diisopropylsebacat, Diisopropyladipat, Dioctylmalat, Dioctylmaleat, Dicaprylylmaleat, Dioctyladipat, Di-n-butyladipat, Diisooctylsuccinat, Di-(2-hexyldecyl)-succinat und Diisotridecylacelaat

Beispiele für erfindungsgemäß besonders bevorzugte Benzoesäureester von linearen oder verzweigten C₁₀-C₂₀-Alkanolen sind unter dem Warenzeichen Finsolv^{®} (Hersteller: Finetex) erhältlich, z. B. Finsolv^{®} TN (Benzoesäure-C12-C15-alkylester), Finsolv^{®} SB (Benzoesäureisostearylester) und Finsolv^{®} BOD (Benzoesäureoctyldocecylester).

Als C₉-C₂₂-Fettalkoholester einwertiger oder mehrwertiger C₂-C₇-Hydroxycarbonsäuren sind die Ester der Glycolsäure, Milchsäure, Äpfelsäure, Weinsäure, Citronensäure und Salicylsäure erfindungsgemäß besonders bevorzugte. Solche Ester auf Basis von linearen C_{14/15}-Alkanolen und von in 2-Position verzweigten C_{12/13}-Alkanolen sind unter dem Warenzeichen Cosmacol^{®} von der Fa. Nordmann, Rassmann GmbH & Co, Hamburg, zu beziehen, Besonders bevorzugt sind die Handelsprodukte Cosmacol^{®} ES), Cosmacol^{®} EMI und Cosmacol^{®} ETI.

Ebenfalls besonders bevorzugte optionale Ölkomponenten sind die Di-n-alkylether wie z. B. Di-n-octylether, Di-(2-ethylhexyl)ether, Laurylmethylether oder Octylbutylether, sowie die Di-n-alkylcarbonate, z. B. Di-n-octylcarbonat.

Weitere bevorzugte Ölkomponenten sind die bei 20 °C noch flüssigen Kohlenwasserstoffe, z. B. Paraffinöle, Isoparaffine und synthetische Kohlenwasserstoffe, z. B. 1,3-Di-(2-ethyl-hexyl)-cyclohexan (Cetiol^{®}S).

Auch bei 20 °C feste Fettkomponenten, wie C14-C30-Fettalkohole, Wachse, beispielsweise Siliconwachse, können optional enthalten sein.

Weitere bevorzugte erfindungsgemäß erhältliche Öl-in-Wasser-Emulsionen sind dadurch gekennzeichnet, dass sie in einem Behälter mit Kugelapplikator oder Roll-on-Applikator verpackt ist

Ein weiterer Gegenstand der vorliegenden Erfindung ist die kosmetische, nicht-therapeutische Verwendung einer Öl-in-Wasser-Emulsion gemäß einem der Ansprüche 27 oder 28, in der der kosmetische Wirkstoff c) ausgewählt ist aus schweißhemmenden Wirkstoffen, zur schweißhemmenden Behandlung der Haut, insbesondere der Achselhaut und/oder der Fußhaut

Ein weiterer Gegenstand der vorliegenden Erfindung ist die kosmetische, nicht-therapeutische Verwendung einer Öl-in-Wasser-Emulsion gemäß einem der Ansprüche 27 oder 28, in der der kosmetische Wirkstoff c) ausgewählt ist aus schweißhemmenden Wirkstoffen, zur schweißhemmenden Behandlung der Haut, insbesondere der Achselhaut und/oder der Fußhaut, mit nicht-fettendem Hautgefühl.

Ein weiterer Gegenstand der vorliegenden Erfindung ist die kosmetische, nicht-therapeutische Verwendung einer Öl-in-Wasser-Emulsion gemäß einem der Ansprüche 27 oder 28, in der der kosmetische Wirkstoff c) ausgewählt ist aus schweißhemmenden Wirkstoffen, zur schweißhemmenden Behandlung der Haut, insbesondere der Achselhaut und/oder der Fußhaut, mit beschleunigter Trocknung.

Ein weiterer Gegenstand der vorliegenden Erfindung ist ein kosmetisches, nicht-therapeutisches Verfahren zur schweißhemmenden Behandlung der Haut, insbesondere der Achselhaut und/oder der Fußhaut, das dadurch gekennzeichnet ist, dass eine Öl-in-Wasser-Emulsion gemäß einem der Ansprüche 27 oder 28, in der der kosmetische Wirkstoff c) ausgewählt ist aus schweißhemmenden Wirkstoffen, in einer wirksamen Menge auf die Haut aufgetragen wird.

Ein weiterer Gegenstand der vorliegenden Erfindung ist die kosmetische, nicht-therapeutische Verwendung einer Öl-in-Wasser-Emulsion gemäß einem der Ansprüche 27 oder 28, in der der kosmetische Wirkstoff c) ausgewählt ist aus desodorierenden Wirkstoffen, Monomeren, Oligomeren und Polymeren von Aminosäuren, N-C₂-C₂₄-Acylaminosäuren, den Estern und/oder den physiologisch verträglichen Salzen dieser Substanzen, DNA- oder RNA-Oligonucleotiden, natürlichen Betainverbindungen, Vitaminen, Provitaminen und Vitaminvorstufen der Gruppen A, B, C, E, H und K und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, Ectoin, Repellentien, anorganischen und organischen UV-Filtersubstanzen, selbstbräunenden Wirkstoffen, hautaufhellenden Wirkstoffen, hautberuhigenden Wirkstoffen, feuchtigkeitsspendenden Wirkstoffen und sebumregulierenden Wirkstoffen, zur desodorierenden, Anti-Falten-, Antiageing-, sebumregulierenden, hautbefeuchtenden bzw. feuchtigkeitsspendenden, insektenabwehrenden, selbstbräunenden oder aufhellenden Behandlung der Haut

Ein weiterer Gegenstand der vorliegenden Erfindung ist die kosmetische, nicht-therapeutische Verwendung einer Öl-in-Wasser-Emulsion gemäß einem der Ansprüche 27 oder 28, in der der kosmetische Wirkstoff c) ausgewählt ist aus desodorierenden Wirkstoffen, Monomeren, Oligomeren und Polymeren von Aminosäuren, N-C₂-C₂₄-Acylaminosäuren, den Estern und/oder den physiologisch verträglichen Salzen dieser Substanzen, DNA- oder RNA-Oligonucleotiden, natürlichen Betainverbindungen, Vitaminen, Provitaminen und Vitaminvorstufen der Gruppen A, B, C, E, H und K und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, Ectoin, Repellentien, anorganischen und organischen UV-Filtersubstanzen, selbstbräunenden Wirkstoffen, hautaufhellenden Wirkstoffen, hautberuhigenden Wirkstoffen, feuchtigkeitsspendenden Wirkstoffen und sebumregulierenden Wirkstoffen, zur desodorierenden, Anti-Falten-, Antiageing-, sebumregulierenden, hautbefeuchtenden bzw. feuchtigkeitsspendenden, insektenabwehrenden, selbstbräunenden oder aufhellenden Hautbehandlung mit nicht-fettendem Hautgefühl.

Ein weiterer Gegenstand der vorliegenden Erfindung ist ein kosmetisches, nicht-therapeutisches Verfahren zur desodorierenden, Anti-Falten-, Antiageing-, sebumregulierenden, hautbefeuchtenden bzw. feuchtigkeitsspendenden, insektenabwehrenden, selbstbräunenden oder aufhellenden Hautbehandlung, das dadurch gekennzeichnet ist, dass eine Öl-in-Wasser-Emulsion gemäß einem der Ansprüche 27 oder 28, in der der kosmetische Wirkstoff c) ausgewählt ist aus desodorierenden Wirkstoffen, Monomeren, Oligomeren und Polymeren von Aminosäuren, N-C₂-C₂₄-Acylaminosäuren, den Estern und/oder den physiologisch verträglichen Salzen dieser Substanzen, DNA- oder RNA-Oligonucleotiden, natürlichen Betainverbindungen, Vitaminen, Provitaminen und Vitaminvorstufen der Gruppen A, B, C, E, H und K und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, Ectoin, Repellentien, anorganischen und organischen UV-Filtersubstanzen, selbstbräunenden Wirkstoffen, hautaufhellenden Wirkstoffen, hautberuhigenden Wirkstoffen, feuchtigkeitsspendenden Wirkstoffen und sebumregulierenden Wirkstoffen, in einer wirksamen Menge auf die Haut aufgetragen wird.

### Erfindungsgemäße Herstellverfahren

Erfindungsgemäß bevorzugte Herstellverfahren sind im folgenden dargestellt.

### Variante 1

Etwa ein Drittel der gesamten Wassermenge (Phase 1) und die Ölkomponente/n i) - iii) und der/die gegebenenfalls enthaltene/n Emulgator/en sowie ggf. weitere Öl- oder Fettkomponenten (Phase 2) werden getrennt voneinander auf eine Temperatur zwischen 70 - 80° C erwärmt. Dann wird Phase 1 langsam zu Phase 2 gegeben und alles 45 - 60 Minuten mit niedriger Drehzahl emulgiert bzw. auf kleiner Stufe homogenisiert. Anschließend wird der Ansatz auf 40 - 45 °C abgekühlt. Anschließend werden der kosmetische Rohstoff c) (sofern dieser ausreichend temperaturstabil ist) und 15 - 20 Gew.-% der gesamten Wassermenge auf 40 - 45 °C erwärmt, zum Ansatz gegeben und homogenisiert. Anschließend wird die noch verbleibende Portion der gesamten Wassermenge zusammen mit dem Polysaccharid und ggf. temperaturempfindlichen Wirkstoffen c) und Zusatzstoffen, beispielsweise Konservierungsmitteln, zum Ansatz gegeben, bei hoher Drehzahl homogenisiert und unter langsamem Rühren auf 25°C abgekühlt.

Die schweißhemmenden Wirkstoffe sind unter den genannten Herstellbedingungen nicht temperaturempfindlich und können bei 40 - 50°C eingearbeitet werden.

Die Dauer der einzelnen Homogenisierschritte beträgt 0,5 - 10 Minuten, bevorzugt 1 - 8 Minuten, besonders bevorzugt 2 - 5 Minuten.

### Verfahren 2

Etwa ein Drittel der gesamten Wassermenge (Phase 1) und die Ölkomponente/n i) - iii) und der/die gegebenenfalls enthaltene/n Emulgator/en sowie ggf. weitere Öl- oder Fettkomponenten (Phase 2) werden getrennt voneinander auf eine Temperatur zwischen 70 - 80° C erwärmt. Dann wird Phase 2 langsam zu Phase 1 gegeben und alles 45 - 60 Minuten mit niedriger Drehzahl emulgiert bzw. auf kleiner Stufe homogenisiert. Anschließend wird der Ansatz auf 40 - 45 °C abgekühlt. Anschließend werden der kosmetische Rohstoff c) (sofern dieser ausreichend temperaturstabil ist) und 15 - 20 Gew.-% der gesamten Wassermenge auf 40 - 45 °C erwärmt, zum Ansatz gegeben und homogenisiert. Anschließend wird die noch verbleibende Portion der gesamten Wassermenge zusammen mit dem Polysaccharid und ggf. temperaturempfindlichen Wirkstoffen c) und Zusatzstoffen, beispielsweise Konservierungsmitteln, zum Ansatz gegeben, bei hoher Drehzahl homogenisiert und unter langsamem Rühren auf 25°C abgekühlt.

Die schweißhemmenden Wirkstoffe sind unter den genannten Herstellbedingungen nicht temperaturempfindlich und können bei 40 - 50°C eingearbeitet werden.

Die Dauer der einzelnen Homogenisierschritte beträgt 0,5 - 10 Minuten, bevorzugt 1 - 8 Minuten, besonders bevorzugt 2 - 5 Minuten.

### Verfahren 3

Etwa 20 % der gesamten Wassermenge werden zusammen mit der/den Ölkomponente/n i) - iii) und den gegebenenfalls enthaltenden Emulgatoren auf eine Temperatur zwischen 70 - 80° C erwärmt und mit niedriger Drehzahl emulgiert bzw. auf kleiner Stufe homogenisiert.

Anschließend werden weitere 10 - 20 % der gesamten Wassermenge auf 70 - 80° C erwärmt, zugefügt, alles bei hoher Drehzahl homogenisiert und anschließend 0,5 - 2 Stunden lang emulgiert. Anschließend werden weitere 10 - 20 % der gesamten Wassermenge auf 70 - 80° C erwärmt, zugefügt und alles bei hoher Drehzahl homogenisiert. Der Ansatz wird auf 40 - 50°C abgekühlt. Eine Portion des kosmetischen Rohstoffs c) wird - sofern dieser Rohstoff bei dieser Temperatur stabil ist - mit weiteren 10 - 20 % der gesamten Wassermenge auf 40 - 50°C erwärmt, zum Ansatz zugegeben und alles bei hoher Drehzahl homogenisiert. Anschließend wird die restliche Portion des kosmetischen Rohstoffs c), sofern dieser Rohstoff bei dieser Temperatur stabil ist, mit weiteren 10 - 20 % der gesamten Wassermenge auf 40 - 50°C erwärmt, zum Ansatz gegeben und alles bei hoher Drehzahl homogenisiert.

Dann wird der Ansatz auf 30 - 35°C abgekühlt. Anschließend wird die noch verbleibende Portion der gesamten Wassermenge zugegeben, bei hoher Drehzahl homogenisiert und langsam unter Rühren abgekühlt.

Falls der kosmetische Rohstoff c) temperaturempfindlich ist, wird er nun erst zusammen mit dem Polysaccharid und ggf. weiteren (temperaturempfindlichen) Zusatzstoffen, beispielsweise Konservierungsmitteln, zum Ansatz gegeben, alles bei hoher Drehzahl homogenisiert und unter langsamem Rühren auf 25°C abgekühlt.

Die schweißhemmenden Wirkstoffe sind unter den genannten Herstellbedingungen nicht temperaturempfindlich und können bei allen Verfahren bei 40 - 50°C eingearbeitet werden.

Die Dauer der einzelnen Homogenisierschritte bei allen Verfahren beträgt 0,5 - 10 Minuten, bevorzugt 1 - 8 Minuten, besonders bevorzugt 2 - 5 Minuten.

Die nachfolgenden Beispiele sollen den Gegenstand der Erfindung näher erläutern, ohne ihn jedoch hierauf zu beschränken.

**Beispiel 1: erfindungsgemäße Antitranspirant-Emulsion (O/W) mit 1,9 Gew.-% Öl- und Fettphase**

| **INGREDIENTS (EU-INCI)** | **Gew.-%-Anteil** | |
|---|---|---|
| AQUA | 81,0 | |
| ALUMINUM CHLOROHYDRATE | 13,0 | |
| STEARETH-2 | 2,5 | |
| STEARETH-21 | 1,5 | |
| PARFUM | 1,1 | Öl |
| PPG-15 STEARYL ETHER | 0,5 | Öl |
| BISABOLOL | 0,1 | Öl |
| ALUMINUM STARCH OCTENYLSUCCINATE | 0,1 | |
| BIS-PEG-18 METHYL ETHER DIMETHYL SILANE | 0,1 | Fett |
| TOCOPHERYL ACETATE | 0,1 | Öl |

Die Emulsion gemäß Beispiel 1 wies am ersten Tag nach der Herstellung eine Viskosität von 1800 mPas auf, gemessen mit einem Brookfield-Viskosimeter, Spindel RV 4, 20 s⁻¹, ohne Helipath, bei 20 °C Umgebungstemperatur und 20 °C Probentemperatur.

**Beispiel 2: erfindungsgemäße Antitranspirant-Emulsion (O/W) mit 1,5 Gew.-% Öl- und Fettphase**

| **INGREDIENTS (EU-INCI)** | **Gew.-%-Anteil** | |
|---|---|---|
| AQUA | 68,3 | |
| ALUMINUM CHLOROHYDRATE | 26,0 | |
| STEARETH-2 | 2,4 | |
| STEARETH-21 | 1,5 | |
| PARFUM | 1,0 | Öl |
| PPG-15 STEARYL ETHER | 0,5 | Öl |
| ALLANTOIN | 0,1 | |
| ALUMINUM STARCH OCTENYLSUCCINATE | 0,1 | |
| ALOE BARBADENSIS | 0,1 | |

Die Emulsion gemäß Beispiel 2 wies am ersten Tag nach der Herstellung eine Viskosität von 2000 mPas auf, gemessen mit einem Brookfield-Viskosimeter, Spindel RV 4, 20 s⁻¹, ohne Helipath, bei 20 °C Umgebungstemperatur und 20 °C Probentemperatur.

**Beispiel 3: erfindungsgemäße Antitranspirant-Emulsion (O/W) mit 2,2 Gew.-% Öl- und Fettphase**

| **INGREDIENTS (EU)** | **Gew.-%-Anteil** | |
|---|---|---|
| AQUA | 90,0 | |
| ALUMINUM ZIRCONIUM TETRACHLOROHYDREX GLY | 23,7 | |
| STEARETH-2 | 2,4 | |
| STEARETH-21 | 1,6 | |
| PARFUM | 1,2 | Öl |
| PPG-15 STEARYL ETHER | 0,5 | Öl |
| ALUMINUM STARCH OCTENYLSUCCINATE | 0,1 | |
| TOCOPHERYL ACETATE | 0,5 | Öl |

Die Emulsion gemäß Beispiel 3 wies am ersten Tag nach der Herstellung eine Viskosität von 2200 mPas auf, gemessen mit einem Brookfield-Viskosimeter, Spindel RV 4, 20 s⁻¹, ohne Helipath, bei 20 °C Umgebungstemperatur und 20 °C Probentemperatur.

**Beispiel 4: erfindungsgemäße Antitranspirant-Emulsion (O/W) mit 1,6 Gew.-% Öl- und Fettphase**

| **INGREDIENTS (EU)** | **Gew.-%-Anteil** | |
|---|---|---|
| AQUA | 74,400000 | |
| ALUMINUM CHLOROHYDRATE | 20,000000 | |
| STEARETH-2 | 2,300000 | |
| STEARETH-21 | 1,500000 | |
| PARFUM | 1,000000 | Öl |
| PPG-15 STEARYL ETHER | 0,500000 | Öl |
| ALUMINUM STARCH OCTENYLSUCCINATE | 0,100000 | |
| ALLANTOIN | 0,100000 | |
| ISOPROPYL MYRISTATE | 0,100000 | Öl |

Die Emulsion gemäß Beispiel 4 wies am ersten Tag nach der Herstellung eine Viskosität von 1700 mPas auf, gemessen mit einem Brookfield-Viskosimeter, Spindel RV 4, 20 s⁻¹, ohne Helipath, bei 20 °C Umgebungstemperatur und 20 °C Probentemperatur.

**Beispiel 5: erfindungsgemäße Antitranspirant-Emulsion (O/W) mit 1,6 Gew.-% Öl- und Fettphase**

| **INGREDIENTS (EU)** | **Gew:-%-Anteil** | |
|---|---|---|
| AQUA | 74,400000 | |
| ALUMINUM CHLOROHYDRATE | 20,000000 | |
| STEARETH-2 | 2,300000 | |
| STEARETH-21 | 1,500000 | |
| PARFUM | 1,000000 | Öl |
| PPG-15 STEARYL ETHER | 0,500000 | Öl |
| DISTARCH PHOSPHATE | 0,100000 | |
| ALLANTOIN | 0,100000 | |
| ISOPROPYL MYRISTATE | 0,100000 | Öl |

Die Emulsion gemäß Beispiel 5 wies am ersten Tag nach der Herstellung eine Viskosität von 1800 mPas auf, gemessen mit einem Brookfield-Viskosimeter, Spindel RV 4, 20 s⁻¹, ohne Helipath, bei 20 °C Umgebungstemperatur und 20 °C Probentemperatur.

Die Emulsionen gemäß den Beispielen 1 - 5 wurden jeweils in eine Flasche mit einem Rollapplikator eingefüllt und waren so verkaufsfertig.

### Lagerstabilität

Alle erfindungsgemäß erhältlichen Beispielemulsionen waren bei 40 °C Lagerung 12 Wochen lang stabil. Alle erfindungsgemäß erhältlichen Beispielemulsionen waren bei 45 °C Lagerung 6 Wochen lang stabil. Bei der Lagerung bei 50 °C zeigte sich je nach Parfümöl eine leichte Aufrahmung zwischen der 3. und 4. Lagerwoche. Ohne die kritischen Parfümöle waren aber auch diese Emulsionen lagerstabil bei 50 °C.

## Patentansprüche

1. Verfahren zur Herstellung einer Öl-in-Wasser-Emulsion, die keine Mikroemulsion darstellt und die enthält:
a) 0,5 - 15 Gew.-% Öl- oder Fettphase, umfassend mindestens eine bei 20 °C flüssige Ölkomponente, ausgewählt aus
i) linearen und verzweigten gesättigten ein- oder mehrwertigen C₃ - C₃₀-Alkanolen, die mit mindestens einer Propylenoxid-Einheit pro Molekül verethert sind,
ii) Propylenglycolmonoestem von verzweigten gesättigten C₆ - C₃₀-Alkancarbonsäuren,
iii) verzweigten gesättigten C₁₀ - C₃₀-Alkanolen,
b) mindestens 60 Gew.-% Wasser,
c) 0,00001 - 38 Gew.-% mindestens eines kosmetischen Wirkstoffes,
d) mindestens ein Polysaccharid in einer Gesamtmenge von 0,01 - 0,2 Gew.-%, bezogen auf das Gesamtgewicht der Emulsion,
e) gegebenenfalls mindestens einen nichtionischen Emulgator mit einem HLB-Wert im Bereich von 3 - 6,
f) gegebenenfalls mindestens einen nichtionischen Emulgator mit einem HLB-Wert im Bereich von 12- 18,
g) mindestens einen Duftstoff in der Öl- oder Fettphase,
h) gegebenenfalls weitere Öl- oder Fettkomponenten,
wobei alle Mengenangaben auf das Gesamtgewicht der Emulsion bezogen sind und das Verfahren folgende Schritte umfasst:
(A) Erwärmung einer Portion X1 der gesamten Wassermenge (Phase X) und der Ölkomponente/n i) - iii) und der/die gegebenenfalls enthaltene/n Emulgator/en sowie ggf. weitere Öl- oder Fettkomponenten (Phase Y) getrennt voneinander auf eine Temperatur zwischen 70 - 80° C,
(B) anschließend langsames Vermischen der Portion X1 von Phase X mit der Phase Y,
(C) anschließend Homogenisierung,
(D) anschließend Rühren bei niedriger Drehzahl im Bereich von 1000 - 2500 Umdrehungen des Rührelementes pro Minute,
(E) anschließend Abkühlen des Ansatzes auf eine Temperatur im Bereich von 40 - 50° C,
(F) anschließend Bereitstellen einer Portion X2 der gesamten Wassermenge, die eine Portion c1 des kosmetischen Wirkstoffs c) enthält, sofern dieser ausreichend temperaturstabil ist, bei einer Temperatur im Bereich von 40 - 60° C,
(G) anschließend langsames Vermischen der Portion (X2 + c1) mit dem gesamten Ansatz bei einer Temperatur im Bereich von 40 - 50° C,
(H) anschließend Homogenisierung,
(I) anschließend langsames Vermischen der noch verbleibenden Portion X3 des Wassers, wobei der Ansatz eine Temperatur im Bereich von 30 - 40° C hat,
(J) anschließend Homogenisierung,
(K) anschließend Zugabe des Polysaccharids und des Duftstoffes sowie ggf. des/der kosmetischen Wirkstoffs/Wirkstoffe c), sofern diese/r bei 40 - 50° C nicht ausreichend temperaturstabil ist/sind sowie ggf. weiterer Zusatzstoffe wie Konservierungsmittel, pH-Regulatoren etc.,
(L) anschließend Homogenisierung,
(M) abschließend Abkühlen auf 25 °C unter langsamem Rühren,
**dadurch gekennzeichnet, dass**
die Verfahrensschritte (F), (G) und (H) einmal, zweimal, dreimal oder mehr als dreimal wiederholt werden, bevor Schritt (I) erfolgt,
die Portion X1 ein Fünftel bis ein Drittel der gesamten Wassermenge beträgt,
der Homogenisierschritt (C) bei niedriger Scherrate im Bereich von 1000 - 2500 Umdrehungen des Rühretementes pro Minute erfolgt und
die Homogenisierschritte (H), (J) und (L) bei hoher Scherrate im Bereich von 3000 - 6000 Umdrehungen des Rührelementes pro Minute erfolgen.

2. Verfahren gemäß Anspruch 1, **dadurch gekennzeichnet, dass** in Schritt (B) die Wasserphase X zur öl- und fetthaltigen Phase (Y) gegeben wird.

3. Verfahren gemäß Anspruch 1, **dadurch gekennzeichnet, dass** in Schritt (B) die öl- und fetthaltige Phase (Y) zur Wasserphase X gegeben wird.

4. Verfahren gemäß einem der vorstehenden Ansprüche, **dadurch gekennzeichnet, dass** das mindestens eine Polysaccharid d) ausgewählt ist aus anionischen und nichtionischen Polysacchariden sowie Mischungen hiervon.

5. Verfahren gemäß Anspruch 4, **dadurch gekennzeichnet, dass** das anionische Polysaccharid ausgewählt Ist aus Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken, Natriumstärkeglycolat sowie Mischungen hiervon.

6. Verfahren gemäß Anspruch 4, **dadurch gekennzeichnet dass** das nichtionische Polysaccharid ausgewählt ist aus Starken, Stärkehydrolysaten, Cellulose, Methylcellulose, Hydroxypropylcellulose, Hydroxyethylcellulose, Hydroxypropylmethylcellulose, Hydroxypropylethylcellulose, Hydroxyethylmethylcellulose sowie Mischungen hiervon.

7. Verfahren gemäß einem der vorstehenden Ansprüche, **dadurch gekennzeichnet, dass** das mindestens eine Polysaccharid in einer Gesamtmenge von 0.05 - 0,2 und bevorzugt 0,09 - 0,2 Gew.-%, jeweils bezogen auf das Gesamtgewicht der Emulsion, enthalten ist.

8. Verfahren gemäß einem der vorstehenden Ansprüche, **dadurch gekennzeichnet, dass** die Ölkomponente i) ausgewählt ist aus Anlagerungsprodukten von mindestens 6 Propylenoxid-Einheiten pro Molekül an ein- oder mehrwertige C₃₋₃₀-Alkanole, insbesondere an Butanol, Butandiol, Myristylalkohol und Stearylalkohol.

9. Verfahren gemäß einem der vorstehenden Ansprüche, **dadurch gekennzeichnet, dass** die Ölkomponente i) ausgewählt ist aus PPG-13-Butylether, PPG-14-Butylether, PPG-9-Butylether, PPG-10-Butandiol und PPG-15-Stearylether sowie Mischungen hiervon.

10. Verfahren gemäß einem der vorstehenden Ansprüche, **dadurch gekennzeichnet, dass** die Ölkomponente ii) ausgewählt ist aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon.

11. Verfahren gemäß einem der vorstehenden Ansprüche, **dadurch gekennzeichnet, dass** die Ölkomponente iii) ausgewählt ist aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon.

12. Verfahren gemäß einem der vorstehenden Ansprüche, **dadurch gekennzeichnet, dass** in der Emulsion mindestens ein nichtionischer Emulgator mit einem HLB-Wert im Bereich von 3 - 6 enthalten ist.

13. Verfahren gemäß Anspruch 12, **dadurch gekennzeichnet, dass** der mindestens eine nichtionische Emulgator mit einem HLB-Wert im Bereich von 3 - 6 ausgewählt ist aus linearen gesättigten und ungesättigten C₁₂ - C₃₀-Alkanolen, die mit 1 - 4 Ethylenoxid-Einheiten pro Molekül verethert sind.

14. Verfahren gemäß Anspruch 12 oder 13, **dadurch gekennzeichnet, dass** der mindestens eine nichtionische Emulgator mit einem HLB-Wert im Bereich von 3 - 6 ausgewählt ist aus Steareth, Ceteth, Myristeth, Laureth, Trideceth, Arachideth und Beheneth mit jeweils 1 - 4 Ethylenoxid-Einheiten pro Molekül.

15. Verfahren gemäß einem der Ansprüche 12 - 14, **dadurch gekennzeichnet, dass** mindestens ein nichtionischer Emulgator mit einem HLB-Wert im Bereich von 3 - 6 in einer Gesamtmenge von 1,8 - 3 Gew.-%, bezogen auf das Gewicht der gesamten Emulsion, enthalten ist.

16. Verfahren gemäß einem der vorstehenden Ansprüche, **dadurch gekennzeichnet, dass** in der Emulsion mindestens ein nichtionischer Emulgator mit einem HLB-Wert im Bereich von 12 - 18 enthalten ist.

17. Verfahren gemäß Anspruch 16, **dadurch gekennzeichnet, dass** der mindestens eine nichtionische Emulgator mit einem HLB-Wert im Bereich von 12 - 18 ausgewählt ist aus linearen gesättigten und ungesättigten C₁₂ - C₂₄-Alkanolen, die mit 7 - 40 Ethylenoxid-Einheiten pro Molekül verethert sind.

18. Verfahren gemäß Anspruch 16 oder 17, **dadurch gekennzeichnet, dass** der mindestens eine nichtionische Emulgator mit einem HLB-Wert im Bereich von 12 - 18 ausgewählt ist aus Steareth, Ceteth, Myristeth, Laureth, Trideceth, Arachideth und Beheneth mit jeweils 7 - 40 Ethylenoxid-Einheiten pro Molekül.

19. Verfahren gemäß einem der vorstehenden Ansprüche, **dadurch gekennzeichnet, dass** in der Emulsion als nichtionischer Emulgator mit einem HLB-Wert im Bereich von 3 - 6 Steareth-2 und gleichzeitig als nichtionischer Emulgator mit einem HLB-Wert im Bereich von 12 - 18 Steareth-21 enthalten ist

20. Verfahren gemäß einem der vorstehenden Ansprüche, **dadurch gekennzeichnet, dass** Steareth-2, Steareth-21 und PPG-15-Stearylether enthalten sind.

21. Verfahren gemäß Anspruch 20, **dadurch gekennzeichnet, dass** mindestens ein Polysaccharid, ausgewählt aus Aluminiumstärkeoctenylsuccinat und Distärkephosphaten, enthalten ist.

22. Verfahren gemäß einem der Ansprüche 16 - 21, **dadurch gekennzeichnet, dass** in der Emulsion mindestens ein nichtionischer Emulgator mit einem HLB-Wert im Bereich von 12 - 18 in einer Gesamtmenge von 1 - 2 Gew.-%, bezogen auf das Gewicht der gesamten Emulsion, enthalten ist.

23. Verfahren gemäß einem der Ansprüche 16 - 22, **dadurch gekennzeichnet, dass** das Gewichtsverhaltnis von nichtionischen Emulgatoren mit einem HLB-Wert im Bereich von 3 - 6 und nichtionischen Emulgatoren mit einem HLB-Wert im Bereich von 12 - 18 von 0,9 bis 3, bevorzugt 1,3 - 1,9 beträgt.

24. Verfahren gemäß einem der vorstehenden Ansprüche, **dadurch gekennzeichnet, dass** in der Emulsion insgesamt maximal 3 Gew.-%, bevorzugt maximal 1 Gew.-% und besonders bevorzugt 0 Gew.-%, jeweils bezogen auf das Gewicht der gesamten Emulsion, an einwertigen C₁ - C₃-Alkanolen, wie Ethanol oder Isopropanol, enthalten ist.

25. Verfahren gemäß einem der vorstehenden Ansprüche, **dadurch gekennzeichnet, dass** der mindestens eine kosmetische Wirkstoff c) ausgewählt ist aus
• schweißhemmenden Wirkstoffen.
• desodorierenden Wirkstoffen,
• Monomeren, Oligomeren und Polymeren von Aminosäuren, N-C₂-C₂₄-Acylaminosäuren, den Estern und/oder den physiologisch verträglichen Salzen dieser Substanzen,
• DNA- oder RNA-Oligonucleotiden,
• natürlichen Betainverbindungen,
• Vitaminen, Provitaminen und Vitaminvorstufen der Gruppen A, B, C, E, H und K und den Estern der vorgenannten Substanzen,
• α-Hydroxycarbonsäuren, α-Ketocarbonsäuren, β-Hydroxycarbonsäuren und deren Ester-, Lacton- oder Salzform,
• Flavonoiden und Flavonoid-reichen Pflanzenextrakten,
• Isoflavonoiden und Isoflavonoid-reichen Pflanzenextrakten,
• Polyphenolen und Polyphenol-reichen Pflanzenextrakten,
• Ubichinon und Ubichinol sowie deren Derivaten,
• Silymarin,
• Ectoin,
• Repelientien,
• anorganischen und organischen UV-Filtersubstanzen,
• selbstbräunenden Wirkstoffen,
• hautaufhellenden Wirkstoffen,
• hautberuhigenden Wirkstoffen,
• feuchtigkeitsspendenden Wirkstoffen,
• sebumregulierenden Wirkstoffen.

26. Verfahren gemäß einem der vorstehenden Ansprüche, **dadurch gekennzeichnet, dass** die Emulsion eine Viskosität Im Bereich von 1000 - 5000 mPas, gemessen 1 Tag nach Herstellung mit einem Brookfield-Viskosimeter, Spindel RV 4, 20 s-1, ohne Helipath, bei 20 °C Umgebungstemperatur und 20 °C Probentemperatur, aufweist.

27. Öl-in-Wasser-Emulsion, erhältlich nach einem Verfahren gemäß einem der vorstehenden Ansprüche.

28. Öl-in-Wasser-Emulsion gemäß Anspruch 27, **dadurch gekennzeichnet, dass** sie in einem Behälter mit Kugelapplikator oder Roll-on-Applikator verpackt ist.

29. Kosmetische, nicht-therapeutische Verwendung einer Öl-in-Wasser-Emulsion gemäß Anspruch 27 oder 28, in der der kosmetische Wirkstoff c) ausgewählt ist aus schweißhemmenden Wirkstoffen, zur schweißhemmenden Behandlung der Haut, insbesondere der Achselhaut und/oder der Fußhaut.

30. Kosmetisches, nicht-therapeutisches Verfahren zur schweißhemmenden Behandlung der Haut, insbesondere der Achselhaut und/oder der Fußhaut, **dadurch gekennzeichnet, dass** eine ÖI-in-Wasser-Emulsion gemäß einem der Ansprüche 27 oder 28, in der der kosmetische Wirkstoff c) ausgewählt Ist aus schweißhemmenden Wirkstoffen, in einer wirksamen Menge auf die Haut aufgetragen wird.

31. Kosmetische, nicht-therapeutische Verwendung einer Öl-in-Wasser-Emulsion gemäß einem der Ansprüche 27 oder 28, in der der kosmetische Wirkstoff c) ausgewählt ist aus desodorierenden Wirkstoffen, Monomeren, Oligomeren und Polymeren von Aminosäuren, N-C₂-C₂₄-Acyl-aminosäuren, den Estern und/oder den physiologisch verträglichen Salzen dieser Substanzen, DNA- oder RNA-Oligonucleotiden, natürlichen Betainverbindungen, Vitaminen, Provitaminen und Vitaminvorstufen der Gruppen A, B, C, E, H und K und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, Ectoin, Repellentien, anorganischen und organischen UV-Filtersubstanzen, salbstbraunenden Wirkstoffen, hautaufhellenden Wirkstoffen, hautberuhigenden Wirkstoffen, feuchtigkeitsspendenden Wirkstoffen und sebumregulierenden Wirkstoffen, zur desodorierenden, Anti-Falten-, Antiageing-, sebumregulierenden, hautbeteuchtenden bzw. feuchtigkeitsspendenden, insektenabwehrenden, selbstbräunenden oder aufhellenden Behandlung der Haut.

## Claims

1. A method for producing an oil-in-water emulsion that does not constitute a microemulsion and contains
a) from 0.5-15 wt.% oily or fatty phase, comprising at least one oil component that is liquid at 20°C, selected from
i) linear and branched saturated monovalent or polyvalent C₃-C₃₀ alkanols that are etherified with at least one propylene oxide unit per molecule,
ii) propylene glycol monoesters of branched saturated C₆-C₃₀ alkane carboxylic acids,
iii) branched saturated C₁₀-C₃₀ alkanols,
b) at least 60 wt.% water,
c) from 0.0001-38 wt.% of at least one cosmetic active ingredient,
d) at least one polysaccharide in a total amount of from 0.01-0.2 wt.%, based on the total weight of the emulsion,
e) optionally at least one non-ionic emulsifier having an HLB value in the range of from 3-6,
f) optionally at least one non-ionic emulsifier having an HLB value in the range of from 12-18,
g) at least one fragrance in the oily or fatty phase,
h) optionally additional oil or fat components,
all the amounts being based on the total weight of the emulsion and the method comprising the following steps:
(A) heating a portion X1 of the total amount of water (phase X) and the oil component(s) i)-iii) and the emulsifier(s) optionally contained and optionally additional oil or fat components (phase Y) separately from one another at a temperature between 70 and 80°C,
(B) then slowly mixing the portion X1 of phase X with phase Y,
(C) subsequent homogenization
(D) then stirring at low speed in the range of from 1000-2500 rpm of the stirring element,
(E) then cooling the batch to a temperature in the range of from 40-50°C,
(F) then providing a portion X2 of the total amount of water, which contains a portion c1 of the cosmetic active ingredient c), if said ingredient is sufficiently thermally stable, at a temperature in the range of from 40-50°C,
(G) then slowly mixing the portion (X2 + c1) with the overall batch at a temperature in the range of from 40-50°C,
(H) subsequent homogenization
(I) then slowly mixing the remaining portion X3 of the water, the batch being at a temperature in the range of from 30-40°C,
(J) subsequent homogenization
(K) then adding the polysaccharide and the fragrance and optionally the cosmetic active ingredient(s) c), if said ingredient(s) is/are not sufficiently thermally stable at 40-50°C, and optionally other additives such as preservatives, pH regulators, etc.,
(L) subsequent homogenization
(M) finally cooling to 25°C with slow stirring,
**characterized in that**
method steps (F), (G) and (H) are repeated once, twice, three times or more than three times before step (I),
the portion X1 is from a fifth to a third of the overall amount of water,
the homogenization step (C) is carried out at a low sheer rate in the range of from 1000-2500 rpm of the stirring element, and
the homogenization steps (H), (J) and (L) are carried out at a high sheer rate in the range of from 3000-6000 rpm of the stirring element.

2. The method according to claim 1, **characterized in that** in step (B) the water phase X is added to the oil- and fat-containing phase (Y).

3. The method according to claim 1, **characterized in that** in step (B) the oil- and fat-containing phase (Y) is added to the water phase X.

4. The method according to one of the preceding claims, **characterized in that** the at least one polysaccharide d) is selected from anionic and non-ionic polysaccharides and mixtures thereof.

5. The method according to claim 4, **characterized in that** the anionic polysaccharide is selected from aluminum starch octenylsuccinate, sodium starch octenylsuccinate, calcium starch octenylsuccinate, distarch phosphates, hydroxyethyl starch phosphates, hydroxypropyl starch phosphates, sodium carboxymethyl starches, sodium starch glycolate, and mixtures thereof.

6. The method according to claim 4, **characterized in that** the non-ionic polysaccharide is selected from starches, starch hydrolysates, cellulose, methylcellulose, hydroxypropyl cellulose, hydroxyethyl cellulose, hydroxypropylmethyl cellulose, hydroxypropylethyl cellulose, hydroxyethylmethyl cellulose, and mixtures thereof.

7. The method according to one of the preceding claims, **characterized in that** the at least one polysaccharide is contained in a total amount of from 0.05-0.2 wt.% and preferably from 0.09-0.2 wt.%, in each case based on the total weight of the emulsion.

8. The method according to one of the preceding claims, **characterized in that** the oil component i) is selected from addition products of at least 6 propylene oxide units per molecule on monovalent or polyvalent C₃₋₃₀ alkanols, in particular on butanol, butanediol, myristyl alcohol and stearyl alcohol.

9. The method according to one of the preceding claims, **characterized in that** the oil component i) is selected from PPG-13 butyl ether, PPG-14 butyl ether, PPG-9 butyl ether, PPG-10 butanediol and PPG-15 stearyl ether, and mixtures thereof.

10. The method according to one of the preceding claims, **characterized in that** the oil component ii) is selected from propylene glycol monoisostearate, propylene glycol monoisopalmitate, propylene glycol monoisobehenate, propylene glycol monoisoarachinate, propylene glycol monoisomyristate, propylene glycol monoisocaprate, propylene glycol monoisocaprinate and propylene glycol monoisocaprylate, and mixtures thereof.

11. The method according to one of the preceding claims, **characterized in that** the oil component iii) is selected from isostearyl alcohol, isocetyl alcohol, isomyristyl alcohol, isotridecyl alcohol, isoarachidyl alcohol, isobehenyl alcohol, isocapryl alcohol, isocaprinyl alcohol, isocaprylyl alcohol, and mixtures thereof.

12. The method according to one of the preceding claims, **characterized in that** a non-ionic emulsifier having an HLB value in the range of from 3-6 is contained in the emulsion.

13. The method according to claim 12, **characterized in that** the at least one non-ionic emulsifier having an HLB value in the range of from 3-6 is selected from linear saturated and unsaturated C₁₂-C₃₀ alkanols etherified with 1-4 ethylene oxide units per molecule.

14. The method according to claim 12 or 13, **characterized in that** the at least one non-ionic emulsifier having an HLB value in the range of from 3-6 is selected from Steareth, Ceteth, Myristeth, Laureth, Trideceth, Arachideth and Beheneth, each having 1-4 ethylene oxide units per molecule.

15. The method according to one of claims 12-14, **characterized in that** at least one non-ionic emulsifier having an HLB value in the range of from 3-6 is contained in a total amount of from 1.8-3 wt.% based on the weight of the overall emulsion.

16. The method according to one of the preceding claims, **characterized in that** at least one non-ionic emulsifier having an HLB value in the range of from 12-18 is contained in the emulsion.

17. The method according to claim 16, **characterized in that** the at least one non-ionic emulsifier having an HLB value in the range of from 12-18 is selected from linear saturated and unsaturated C₁₂-C₂₄ alkanols etherified with 7-40 ethylene oxide units per molecule.

18. The method according to claim 16 or 17, **characterized in that** the at least one non-ionic emulsifier having an HLB value in the range of from 12-18 is selected from Steareth, Ceteth, Myristeth, Laureth, Trideceth, Arachideth and Beheneth, each having 7-40 ethylene oxide units per molecule.

19. The method according to one of the preceding claims, **characterized in that** Steareth-2 is contained in the emulsion as the non-ionic emulsifier having an HLB value in the range of from 3-6 and at the same time Steareth-21 is contained as the non-ionic emulsifier having an HLB value in the range of from 12-18.

20. The method according to one of the preceding claims, **characterized in that** Steareth-2, Steareth-21 and PPG-15 stearyl ether are contained.

21. The method according to claim 20, **characterized in that** at least one polysaccharide is contained, selected from aluminum starch octenylsuccinate and distarch phosphates.

22. The method according to one of claims 16-21, **characterized in that** at least one non-ionic emulsifier having an HLB value in the range of from 12-18 is contained in the emulsion in a total amount of from 1-2 wt.% based on the weight of the overall emulsion.

23. The method according to one of claims 16-22, **characterized in that** the weight ratio of non-ionic emulsifiers having an HLB value in the range of from 3-6 and non-ionic emulsifiers having an HLB value in the range of from 12-18 is from 0.9-3, preferably from 1.3-1.9.

24. The method according to one of the preceding claims, **characterized in that** the emulsion contains a total of at most 3 wt.%, preferably at most 1 wt.%, and particularly preferably 0 wt.%, in each case based on the weight of the overall emulsion, of monovalent C₁-C₃ alkanols such as ethanol or isopropanol.

25. The method according to one of the preceding claims, **characterized in that** the at least one cosmetic active ingredient c) is selected from
• antiperspirant active ingredients,
• deodorizing active ingredients,
• monomers, oligomers and polymers of amino acids, N-C₂-C₂₄ acyl amino acids, the esters and/or physiologically acceptable salts of these substances,
• DNA or RNA oligonucleotides,
• natural betaine compounds,
• vitamins, provitamins and vitamin precursors of the groups A, B, C, E, H and K and the esters of said substances,
• α-hydroxycarboxylic acids, α-ketocarboxylic acids, β-hydroxycarboxylic acids and the ester, lactone or salt form thereof,
• flavonoids and flavonoid-rich plant extracts,
• isoflavonoids and isoflavonoid-rich plant extracts,
• polyphenols and polyphenol-rich plant extracts,
• ubiquinone and ubiquinol and the derivatives thereof,
• silymarin,
• ectoine,
• repellents,
• inorganic and organic UV filter substances,
• self-tanning active ingredients,
• skin-lightening active ingredients,
• skin-soothing active ingredients,
• moisturizing active ingredients,
• sebum-regulating active ingredients.

26. The method according to one of the preceding claims, **characterized in that** the emulsion has a viscosity in the range of from 1000-5000 mPa, measured 1 day after production using a Brookfield viscometer, spindle RV-4, 20 s-1, without a Helipath, at 20°C ambient temperature and 20°C sample temperature.

27. An oil-in-water emulsion that can be obtained by a method according to one of the preceding claims.

28. The oil-in-water emulsion according to claim 27, **characterized in that** it is packaged in a container having a ball applicator or roll-on applicator.

29. The cosmetic, non-therapeutic use of an oil-in-water emulsion according to claim 27 or 28 in which the cosmetic active ingredient c) is selected from antiperspirant active ingredients, for anti-perspiration treatment of the skin, in particular the skin of the armpit and/or of the feet.

30. A cosmetic, non-therapeutic method for the anti-perspiration treatment of the skin, in particular the skin of the armpit and/or of the feet, **characterized in that** an oil-in-water emulsion according to one of claims 27 or 28, in which the cosmetic active ingredient c) is selected from antiperspirant active ingredients, is applied to the skin in an effective amount.

31. The cosmetic, non-therapeutic use of an oil-in-water emulsion according to one of claims 27 or 28 in which the cosmetic active ingredient c) is selected from deodorizing active ingredients, monomers, oligomers and polymers of amino acids, N-C₂-C₂₄ acyl amino acids, the esters and/or physiologically acceptable salts of these substances, DNA or RNA oligonucleotides, natural betaine compounds, vitamins, provitamins and vitamin precursors of the groups A, B, C, E, H and K and the esters of said substances, α-hydroxycarboxylic acids, α-ketocarboxylic acids, β-hydroxycarboxylic acids and the ester, lactone or salt form thereof, flavonoids and flavonoid-rich plant extracts, isoflavonoids and isoflavonoid-rich plant extracts, polyphenols and polyphenol-rich plant extracts, ubiquinone and ubiquinol and the derivatives thereof, silymarin, ectoine, repellents, inorganic and organic UV filter substances, self-tanning active ingredients, skin-lightening active ingredients, skin-soothing active ingredients, moisturizing active ingredients and sebum-regulating active ingredients, for the deodorizing, anti-wrinkle, anti-aging, sebum-regulating, skin-moisturizing or moisturizing, insect-repellent, self-tanning or skin-lightening treatment of the skin.

## Revendications

1. Procédé de préparation d'une émulsion huile-dans-eau qui n'est pas une microémulsion et qui contient
a) 0,5 à 15% en poids de phase huileuse ou graisseuse comportant au moins un composant huileux liquide à 20°C, choisi parmi
i) les alcanols en C₃ à C₃₀ mono- ou polyvalents saturés, linéaires ou ramifiés, qui sont éthérifiés avec au moins une unité d'oxyde de propylène par molécule,
ii) les monoesters de propylène-glycol d'acide d'alcane carbonique en C₆ à C₃₀ saturés ramifiés,
iii) les alcanols en C₁₀ à C₃₀ saturés ramifiés,
b) au moins 60% en poids d'eau,
c) 0,00001 à 38% en poids d'au moins une substance active cosmétique,
d) au moins un polysaccharide dans une quantité totale de 0,01 à 0,2% en poids, sur la base du poids total de l'émulsion,
e) éventuellement au moins un émulsifiant non-ionique ayant une valeur HLB dans la gamme de 3 à 6,
f) éventuellement au moins un émulsifiant non-ionique ayant une valeur HLB dans la gamme de 12 à 18,
g) au moins une substance odorante dans la phase huileuse ou graisseuse,
h) éventuellement d'autres composants huileux ou graisseux,
toutes les données quantitatives se rapportant au poids total de l'émulsion et le procédé comprenant les étapes suivantes consistant à
(A) chauffer une partie X1 de la toute quantité d'eau (phase X) et le ou les composants huileux i) à iii) et/ou le ou les émulsifiant éventuellement contenus ainsi qu'éventuellement d'autres huileux ou graisseux (phase Y) séparément les uns des autres à une température comprise entre 70 et 80°C,
(B) puis mélanger lentement la partie X1 de la phase X avec la phase Y,
(C) puis homogénéiser,
(D) puis agiter à faible vitesse dans la gamme de 1000 à 2500 tours de l'élément d'agitation par minute,
(E) puis refroidir le dépôt à une température dans la gamme de 40 à 50°C,
(F) puis produire une partie X2 de toute la quantité d'eau qui contient une partie c1 de la substance active cosmétique c) à condition que celle-ci soit suffisamment stabile en température, à une température dans la gamme de 40 à 50°C,
(G) puis mélanger lentement la partie (X2+c1) avec tout le dépôt à une température dans la gamme de 40 à 50°C,
(H) puis homogénéiser,
(I) puis mélanger lentement la partie encore restante X3 de l'eau, le dépôt ayant une température dans la gamme de 30 à 40°C,
(J) puis homogénéiser,
(K) puis ajouter le polysaccharide et la substance odorante et éventuellement le ou les substances actives cosmétique c) à condition qu'elles soient insuffisamment stables à la température de 40 à 50°C et éventuellement d'autres additifs tels que des conservateurs, des régulateurs de pH, etc.
(L) puis homogénéiser,
(M) puis refroidir à 25°C avec agitation lente,
**caractérisé en ce que**
les étapes de procédé (F), (G) et (H) sont réalisées de façon répétée une fois, deux fois, trois fois ou plus de trois fois avant l'étape (i),
la partie X1 représente un cinquième à un tiers de toute la quantité d'eau,
l'étape d'homogénéisation (C) est effectuée à faible gradient de cisaillement dans la gamme de 1000 à 2500 tours de l'élément d'agitation par minute et
les étapes d'homogénéisation (H), (J) et (L) sont effectuées à un gradient de cisaillement élevé dans la gamme de 3000 à 6000 tours de l'élément d'agitation par minute.

2. Procédé selon la revendication 1, **caractérisé en ce qu'**à l'étape (B) la phase aqueuse X est ajoutée à la phase huileuse et graisseuse (Y).

3. Procédé selon la revendication 1, **caractérisé en ce qu'**à l'étape (B) la phase huileuse et graisseuse (Y) est ajoutée à la phase aqueuse X.

4. Procédé selon l'une des revendications précédentes, **caractérisé en ce que** l'moins un polysaccharide d) est choisi parmi les polysaccharides anioniques et non-ioniques et leurs mélanges.

5. Procédé selon la revendication 4, **caractérisé en ce que** le polysaccharide anionique est choisi parmi l'octénylsuccinate d'amidon aluminique, l'octénylsuccinate d'amidon sodique, l'octénylsuccinate d'amidon calcique, les diphosphates d'amidon, les phosphates d'hydroxyéthylamidons, les phosphates d'hydroxypropylamidons, les carboxyméthylamidons sodiques, les glycolates d'amidons sodiques et leurs mélanges.

6. Procédé selon la revendication 4, **caractérisé en ce que** le polysaccharide non-ionique est choisi parmi les amidons, les hydrolysats d'amidons, la cellulose, la méthylcellulose, l'hydroxypropylcellulose, l'hydroxyethylcellulose, l'hydroxypropylméthylcellulose, l'hydroxypropyléthylcellulose, l'hydroxyéthylméthylcellulose et leurs mélanges.

7. Procédé selon l'une des revendications précédentes, **caractérisé en ce que** l'au moins un polysaccharide est contenu dans une quantité totale de 0,05 à 0,2% en poids et de préférence de 0,09 à 0,2% en poids, à chaque fois par rapport au poids total de l'émulsion.

8. Procédé selon l'une des revendications précédentes, **caractérisé en ce que** le composant huileux i) est choisi parmi les produits d'addition d'au moins 6 unités d'oxyde de propylène par molécule à des alcanols en C₃ à C₃₀ mono- ou polyvalents, en particulier au butanol, au butanediol, à l'alcool myristylique et à l'alcool stéarylique.

9. Procédé selon l'une des revendications précédentes, **caractérisé en ce que** le composant huileux i) est choisi le PPG-13-butyléther, PPG-14-butyléther, PPG-9-butyléther, le PPG-10-butanediol et le PPG-15-éther stéarylique et leurs mélanges.

10. Procédé selon l'une des revendications précédentes, **caractérisé en ce que** le composant huile ii) est choisi parmi le monoisostéarate de propylène-glycol, le monoisopalmitate de propylène-glycol, le monoisobéhénat de propylène-glycol, le monoisoarachinate de propylène-glycol, le monoisomyristate de propylène-glycol, le monoisocaprate de propylène-glycol, le monoisocaprinate de propylène-glycol et le monoisocaprylate de propylène-glycol et leurs mélanges.

11. Procédé selon l'une des revendications précédentes, **caractérisé en ce que** le composant huileux iii) est choisi parmi l'alcool isostéarylique, l'alcool isocétylique, l'alcool isomyristylique, l'alcool isotridécylique, l'alcool isoarachidylique, l'alcool isobéhénylique, l'alcool isocaprylique, l'alcool isocaprinylique, l'alcool isocaprylque et leurs mélanges.

12. Procédé selon l'une des revendications précédente, **caractérisé en ce que** l'émulsion contient au moins un émulsifiant non-ionique ayant une valeur HLB dans la gamme de 3 à 6.

13. Procédé selon la revendication 12, **caractérisé en ce que** l'au moins un émulsifiant non-ionique ayant une valeur HLB dans la gamme de 3 à 6 est choisi parmi les alcanols en C₁₂ à C₃₀ linéaires saturés et insaturées qui sont éthérifiés avec 1 à 4 unités d'oxyde d'éthylène par molécule.

14. Procédé selon la revendication 12 ou 13, **caractérisé en ce que** l'au moins un émulsifiant non-ionique ayant une valeur HLB dans la gamme de 3 à 6 est sélectionné parmi le Stéareth, le Céteth, le Myristeth, le Laureth, le Tridéceth, l'Arachideth et le Béhéneth comportant chacun 1 à 4 unités d'oxyde d'éthylène par molécule.

15. Procédé selon l'une des revendications 12 à 14, **caractérisé en ce qu'**au moins un émulsifiant non-ionique ayant une valeur HLB dans la gamme de 3 à 6 est contenu dans une quantité totale de 1,8 à 3% en poids, par rapport au poids de toute l'émulsion.

16. Procédé selon l'une des revendications précédentes, **caractérisé en ce que** l'émulsion contient au moins un émulsifiant non-ionique ayant une valeur HLB dans la gamme de 12 à 18.

17. Procédé selon la revendication 16, **caractérisé en ce que** l'au moins un émulsifiant non-ionique ayant une valeur HLB dans la gamme de 12 à 18 est choisi parmi les alcanols C₁₂ à C₂₄ linéaires saturés et insaturés, qui sont éthérifiés avec 7 à 40 unités d'oxyde d'éthylène par molécule.

18. Procédé selon la revendication 16 ou 17, **caractérisé en ce que** l'au moins un émulsifiant non-ionique ayant une valeur HLB dans la gamme de 12 à 18 est choisi parmi le Stéareth, le Céteth, le Myristeth, le Laureth, le Tridéceth, l'Arachideth et le Béhéneth comportant chacun 7 à 40 unités d'oxyde d'éthylène par molécule.

19. Procédé selon l'une des revendications précédentes, **caractérisé en ce que** l'émulsion contient comme émulsifiant non-ionique ayant une valeur HLB dans la gamme de 3 à 6 le Stéareth-2 et en même temps comme émulsifiant non-ionique ayant une valeur HLB dans la gamme de 12 à 18 le Stéareth-21

20. Procédé selon l'une des revendications précédentes, **caractérisé en ce que** le Stéareth-2, le stéareth-21 et le PPG-15-éther stéarylique sont contenus.

21. Procédé selon la revendication 20, **caractérisé en ce qu'**au moins un polysaccharide est contenu qui est choisi parmi l'octénylsuccinate d'amidon aluminique et les diphosphates d'amidons.

22. Procédé selon l'une des revendications 16 à 21, **caractérisé en ce que** l'émulsion contient au moins un émulsifiant non-ionique ayant une valeur HLB dans la gamme de 12 à 18 dans une quantité totale de 1 à 2% en poids, par rapport au poids de toute l'émulsion.

23. Procédé selon l'une des revendications précédentes 16 à 22, **caractérisé en ce que** le rapport en poids des émulsifiants non-ioniques ayant une valeur HLB dans la gamme de 3 à 6 et des émulsifiants non-ioniques ayant une valeur HLB dans la gamme de 12 à 18 est 0,9 à 3, de préférence de 1,3 à 1,9.

24. Procédé selon l'une des revendications précédentes, **caractérisé en ce que** l'émulsion contient au total un maximum de 3% en poids, de préférence un maximum de 1% en poids et de façon particulièrement préféré de 0% en poids, par rapport au poids de toute l'émulsion, d'alcanols en C₁ à C₃ monovalents tels que l'éthanol ou l'isapropanol.

25. Procédé selon l'une des revendications précédentes, **caractérisé en ce que** l'au moins une substance active cosmétique c) est choisie parmi
• les substances actives anti-transpirantes,
• les substances actives désodorisantes,
• les monomères, les oligomères et les polymères d'acides aminés, les acides N-acylaminés en C₂ à C₂₄, les esters et/ou les sels physiologiquement acceptables de ces substances,
• les oligonucléotides d'ADN ou d'ARN,
• les composés naturels de la bétaïne,
• les vitamines, les provitamines et les précurseurs de vitamines des groupes A, B, C, E, H et K et les esters des substances susmentionnées,
• les acides α-hydroxycarboxyliques, les α-cétocarboxyliques, les acides β-hydroxycarboxyliques et leur forme ester, lactone, ou sel,
• les flavonoïdes et les extraits végétaux riches en flavonoïdes,
• les isoflavonoïdes et les extraits végétaux riches en isoflavonoïdes,
• les polyphénols et les extraits végétaux riches en polyphénols,
• l'ubiquinone et l'ubichinol et leurs dérivés,
• la silymarine,
• l'ectoïne,
• les répulsifs,
• les substances minérales et organiques filtrant les UV,
• les substances actives auto-bronzantes
• les substances actives d'éclaircissement de la peau,
• les substances actives apaisantes,
• les substances actives hydratantes,
• les substances actives régulatrices de sébum.

26. Procédé selon l'une des revendications précédentes, **caractérisé en ce que** l'émulsion a une viscosité dans la gamme de 1000 à 5000 mPas, mesurée 1 jour après la production avec un viscosimètre Brookfield RV broche 4, 20 s-1, sans Hélipath, à une température ambiante de 20°C et une température d'échantillon de 20°C.

27. Émulsion Huile-dans-eau, pouvant être obtenue par un procédé selon l'une des revendications précédentes.

28. Émulsion huile-dans-eau selon la revendication 27, **caractérisée en ce qu'**elle est conditionnée dans un récipient comportant un applicateur à boule ou un applicateur Roll-on.

29. Utilisation cosmétique non-thérapeutique d'une émulsion huile-dans-eau selon la revendication 27 ou 28, dans laquelle la substance active cosmétique est choisie parmi les substances actives anti-transpirantes pour le traitement anti-transpirant de la peau, en particulier de la peau des aisselles et/ou de la peau des pieds.

30. Procédé cosmétique non-thérapeutique de traitement anti-transpirant de la peau, en particulier de la peau des aisselles et/ou de la peau des pieds, **caractérisé en ce qu'**une émulsion huile-dans-eau selon l'une des revendications 27 ou 28, dans laquelle la substance active cosmétique substance c) est choisie parmi les substances actives anti-transpirantes et est appliquée pour la peau dans une quantité efficace.

31. Utilisation cosmétique non-thérapeutique d'une émulsion huile-dans-eau selon l'une des revendications 27 ou 28, dans laquelle la substance active cosmétique est choisie parmi les substances actives désodorisantes, les monomères, les oligomères et les polymères d'acides aminés, les acides N-acylaminés en C₂ à C₂₄, les esters et/ou les sels physiologiquement acceptables de ces substances, les oligonucléotides d'ADN ou d'ARN, les composés naturels de la bétaïne, les vitamines, les provitamines et les précurseurs de vitamines des groupes A, B, C, E, H et K et les esters des substances susmentionnées, les acides α-hydroxycarboxyliques, les α-cétocarboxyliques, les acides β-hydroxycarboxyliques et leur forme ester, lactone, ou sel, les flavonoïdes et les extraits végétaux riches en flavonoïdes, les isoflavonoïdes et les extraits végétaux riches en isoflavonoïdes, les polyphénols et les extraits végétaux riches en polyphénols, l'ubiquinone et l'ubichinol et leurs dérivés, la silymarine, l'ectoïne, les répulsifs, les substances minérales et organiques filtrant les UV, les substances actives auto-bronzantes, les substances actives d'éclaircissement de la peau, les substances actives apaisantes, les substances actives hydratantes, les substances actives régulatrices de sébum, pour le traitement désodorisant, antirides, anti-âge, régulateur de sébum, humectant ou hydratant, ou répulsifs d'insectes, auto-bronzant ou éclaircissant.
